# EUROPEAN PATENT SPECIFICATION

(11) **EP 1 149 579 B1**
(45) Date of publication and mention of the grant of the patent: **06.12.2006**
(21) Application number: 01303481.4
(22) Date of filing: 12.04.2001
(51) Int. Cl.: A61K 31/00, A61K 31/4453, A61K 31/40, A61K 31/4439, A61K 31/4725, A61K 31/138, A61K 31/4535, A61K 31/453, A61K 31/55, A61K 31/404, A61P 15/00, A61P 15/02

(54) **Use of an estrogen agonist/antagonist for treating female sexual dysfunction**
Verwendung von einem Östrogen Agonist/Antagonist zur Behandlung der weiblichen sexuellen Störungen
Utilisation d'un agoniste/antagoniste estrogénique pour traiter le dysfonctionnement sexuel chez la femme

(30) Priority: 18.04.2000 US 266387 P
(43) Date of publication of application: 31.10.2001
(73) Proprietor: Pfizer Products Inc., Groton, Connecticut 06340 (US)
(72) Inventor: Lee, Andrew George, Pfizer Global, Groton, Connecticut 06340 (US); Thompson, David Duane, Pfizer Global, Groton, Connecticut 06340 (US); Day, Wesley Warren, Pfizer Global, Groton, Connecticut 06340 (US)
(74) Representative: Rutt, Jason Edward

(56) References cited:
- EP-A- 0 792 641

## Description

### FIELD OF THE INVENTION

This invention relates to pharmaceutical compositions for the treatment of female sexual dysfunction comprising estrogen agonists / antagonists and pharmaceutically acceptable salts thereof, kits containing such compositions and methods of using estrogen agonists / antagonists to treat female sexual dysfunction. Optionally, the compositions, kits and methods of the invention may further include or utilize a cyclic guanosine 3',5'-monophosphate elevator compound.

### BACKGROUND OF THE INVENTION

Female sexual dysfunction (FSD) includes hypoactive sexual desire disorder, sexual anhedonia and dyspareunia. Proper sexual functioning in women depends on the sexual response cycle, which consists of an anticipatory mental set (sexual motive state or state of desire), effective vasocongestive arousal (swelling and lubrication), orgasm, and resolution. In women, orgasm is accompanied by contractions (not always subjectively experienced as such) of the muscles of the outer third of the vagina. Generalized muscular tension, perineal contractions, and involuntary pelvic thrusting (every 0.8 sec) usually occur. Orgasm is followed by resolution-a sense of general pleasure, well-being, and muscular relaxation. During this phase, women may be able to respond to additional stimulation almost immediately.

The sexual response cycle is mediated by a delicate, balanced interplay between the sympathetic and parasympathetic nervous systems. Vasocongestion is largely mediated by parasympathetic (cholinergic) outflow; orgasm is predominantly sympathetic (adrenergic). These responses are easily inhibited by cortical influences or by impaired hormonal, neural, or vascular mechanisms. Disorders of sexual response may involve one or more of the cycle's phases. Generally, both the subjective components of desire, arousal, and pleasure and the objective components of performance, vasocongestion, and orgasm are disturbed, although any may be affected independently. Sexual dysfunctions may be lifelong (no effective performance ever, generally due to intrapsychic conflicts) or acquired (after a period of normal function); generalized or limited to certain situations or certain partners; and total or partial.

Hypoactive sexual desire disorder is a disorder in which sexual fantasies and desire for sexual activity are persistently or recurrently diminished or absent, causing marked distress or interpersonal difficulties. Hypoactive sexual desire disorder may be lifelong or acquired, generalized (global) or situational (partner-specific). Sexual desire is a complex psychosomatic process based on brain activity (the "generator" or "motor" running in a rheostatic cyclic fashion), a poorly defined hormonal milieu, and cognitive scripting that includes sexual aspiration and motivation. Desynchronization of these components results in hypoactive sexual desire disorder.

The acquired form of hypoactive sexual desire disorder is commonly caused by boredom or unhappiness in a long-standing relationship, depression (which leads more often to decreased interest in sex than it does to impotence in the male or to inhibited excitement in the female), dependence on alcohol or psychoactive drugs, side effects from prescription drugs (e.g., antihypertensives, antidepressants), and hormonal deficiencies. This disorder can be secondary to impaired sexual functioning in the arousal or orgasm phase of the sexual response cycle.

Symptoms and signs of hypoactive sexual desire disorder include the patient complaining of a lack of interest in sex, even in ordinarily erotic situations. The disorder is usually associated with infrequent sexual activity, often causing serious marital conflict. Some patients have sexual encounters fairly often to please their partners and may have no difficulty with performance but continue to have sexual apathy. When boredom is the cause, frequency of sex with the usual partner decreases, but sexual desire may be normal or even intense with others (the situational form).

Clinically significant sexual dysfunction that causes personal distress or interpersonal problems and is most likely fully explained by direct physiologic effects of a physical disorder. Sexual dysfunction due to a physical disorder is usually generalized (not specific to a given partner or situation). It is diagnosed when evidence from a patient's history, physical examination, or laboratory assessment can explain the dysfunction physiologically and when mental disorders that may better explain it can be ruled out. Resolution of the underlying physical disorders often results in resolution or amelioration of the sexual dysfunction. When the cause of sexual dysfunction is a combination of psychologic and physical factors, the appropriate diagnosis is sexual dysfunction due to combined factors.

Sexual anhedonia (decreased or absent pleasure in sexual activity) is not an official diagnosis. It is almost always classified under hypoactive sexual desire disorder, because loss of pleasure almost always results in loss of desire (although loss of desire may occur first). The cause is likely to be depression or drugs if anhedonia is acquired and global (with all partners in all situations); interpersonal factors if anhedonia is confined to one partner or one situation; or repressive factors (e.g., guilt, shame) due to family dysfunction or childhood trauma if anhedonia is lifelong. Sexual aversion is the probable diagnosis in lifelong cases.

Sexual arousal disorder is the persistent or recurrent inability to attain or to maintain the lubrication-swelling response of sexual excitement until completion of sexual activity. This disturbance occurs despite adequate focus, intensity, and duration of sexual stimulation. The disorder may be lifelong or, more commonly, acquired and restricted to the partner. The patient's complaints are usually related to lack of orgasm, although some women report lack of excitement.

Although women can be orgasmic throughout their lives, sexual activity often decreases after age 60 because of the relative lack of partners and untreated physiologic changes (e.g., atrophy of the vaginal mucosa, with resultant dryness and painful coitus).

The female sexual response phase of arousal is not easily distinguished from the phase of desire until physiological changes begin to take place in the vagina and clitoris as well as other sexual organs. Sexual excitement and pleasure are accompanied by a combination of vascular and neuromuscular events which lead to engorgement of the clitoris, labia and vaginal wall, increased vaginal lubrication and dilatation of the vaginal lumen (Levin, R.J., Clin. Obstet. Gynecol., 1980:7; 213-252; Ottesen, B., Gerstenberg, T., Ulrichsen, H. et al., Eur. J. Clin. Invest., 1983:13; 321-324; Levin, R.J., Exp. Clin. Endocrinol., 1991:98; 61-69; Levin, R.J., Ann. Rev. Sex Res., 1992:3; 1-48; Masters, W. H., Johnson, V. E. Human Sexual Response. Little, Brown: Boston, 1996; Berman, J.R., Berman, L. & Goldstein, L., Urology, 1999:54; 385-391).

Vaginal engorgement enables transudation to occur and this process is responsible for increased vaginal lubrication. Transudation allows a flow of plasma through the epithelium and onto the vaginal surface, the driving force for which is increased blood flow in the vaginal capillary bed during the aroused state. In addition engorgement leads to an increase in vaginal length and luminal diameter, especially in the distal 2/3 of the vaginal canal. The luminal dilatation of the vagina is due to a combination of smooth muscle relaxation of its wall and skeletal muscle relaxation of the pelvic floor muscles. Some sexual pain disorders such as vaginismus are thought to be due, at least in part, by inadequate relaxation preventing dilatation of the vagina; it has yet to be ascertained if this is primarily a smooth or skeletal muscle problem. (Masters, W. H., Johnson, V. E. Human Sexual Response. Little, Brown: Boston, 1996; Berman, J.R., Berman, L. & Goldstein, L., Urology, 1999:54; 385-391).

The categories of FSD are best defined by contrasting them to the phases of normal female sexual response: desire, arousal and orgasm. Desire or libido is the drive for sexual expression. Its manifestations often include sexual thoughts either when in the company of an interested partner or when exposed to other erotic stimuli. Arousal is the vascular response to sexual stimulation, an important component of which is vaginal lubrication and elongation of the vagina. Orgasm is the release of sexual tension that has culminated during arousal.

Hence, FSD occurs when a woman has an inadequate or unsatisfactory response in any of these phases; desire, arousal or orgasm. FSD categories include hypoactive sexual desire disorder, sexual arousal disorder, orgasmic disorders and sexual pain disorders.

Hypoactive sexual desire disorder is present if a woman has no or little desire to be sexual, and has no or few sexual thoughts or fantasies. This type of FSD can be caused by low testosterone levels, due either to natural menopause or to surgical menopause. Other causes include illness, medications, fatigue, depression and anxiety.

Sexual arousal disorder (FSAD) is characterized by inadequate genital response to sexual stimulation. The genitalia do not undergo the engorgement that characterizes normal sexual arousal. The vaginal walls are poorly lubricated, so that intercourse is painful. Orgasms may be impeded. Arousal disorder can be caused by reduced estrogen at menopause or after childbirth and during lactation, as well as by illnesses, with vascular components such as diabetes and atherosclerosis. Other causes result from treatment with diuretics, antihistamines, antidepressants, e.g., SSRIs or antihypertensive agents.

Sexual pain disorders (includes dyspareunia and vaginismus) is characterized by pain resulting from penetration and may be caused by medications which reduce lubrication, endometriosis, pelvic inflammatory disease, inflammatory bowel disease or urinary tract problems.

Dyspareunia is painful coitus or attempted coitus. Dyspareunia is usually introital but may also occur before, during, or after intercourse. Causes include menopausal involution with dryness and thinning of the mucosa. Pain during or after coitus is the chief complaint.

The prevalence of FSD is difficult to gauge because the term covers several types of problems, some of which are difficult to measure, and because the interest in treating FSD is relatively recent. Many women's sexual problems are associated either directly with the female aging process or with chronic illnesses such as diabetes or hypertension.

There are wide variations in the reported incidence and prevalence of FSD, in part explained by the use of differing evaluation criteria, but most investigators report that a significant proportion of otherwise healthy women have symptoms of one or more of the FSD subgroups. By way of example, studies comparing sexual dysfunction in couples reveal that 63% of women had arousal or orgasmic dysfunction compared with 40% of men having erectile or ejaculatory dysfunction (Frank, E., Anderson, C. & Rubinstein, D., N. Engl. J. Med., 11:229;111-115). However, the prevalence of female sexual arousal disorder varies considerably from survey to survey. In a recent National Health and Social Life Survey, 19% of women reported lubrication difficulties whereas 14% of women in an outpatient gynecological clinic reported similar difficulties with lubrication (Rosen, R., Taylor, J., Leiblum, S. et al., J. Sex Marital Ther., 1993:19; 171-188).

Several studies have also reported dysfunction with sexual arousal in diabetic women (up to 47%), including reduced vaginal lubrication (Wincze, J.P., Albert, A. & Bansal, S., Arch. Sex Behav., 1993:22; 587-601). There was no association between neuropathy and sexual dysfunction. Numerous studies have also shown that between 11-48% of women overall may have reduced sexual desire with age. Similarly, between 11-50% of women report problems with arousal and lubrication, and therefore experience pain with intercourse. Vaginismus is far less common, affecting approximately 1 % of women. Studies of sexually experienced women have detailed that 5-10% have primary anorgasmia. Another 10% have infrequent orgasms and a further 10% experience them inconsistently (Spector, I.P. & Carey, M.P., Arch. Sex. Behav., 1990:19; 389-408).

FSAD is a highly prevalent sexual disorder affecting pre-, peri- and post menopausal (±HRT) women. It is associated with concomitant disorders such as depression, cardiovascular diseases, diabetes and UG disorders. The primary consequences of FSAD are lack of engorgement/swelling, lack of lubrication and lack of pleasurable genital sensation. The secondary consequences of FSAD are reduced sexual desire, pain during intercourse and difficulty in achieving an orgasm. It has recently been hypothesized that there is a vascular basis for at least a proportion of patients with symptoms of FSAD (Goldstein, L. & Berman, J.R., Int. J. Impot. Res., 1998:10; S84-S90) with animal data supporting this view (Park, K., Goldstein, I., Andry, C., et al., Int. J. Impotence Res., 1997:9; 27-37).

The hormone estrogen has a profound effect in the vascular system of both men and women although its administration is associated with other effects that can be undesirable. Estrogen increases vasodilatation and inhibits the response of blood vessels to injury and the development of atherosclerosis. Estrogen-induced vasodilatation occurs 5 to 20 minutes after estrogen has been administered and is not dependent on changes in gene expression; this action of estrogen is sometimes referred to as "nongenomic." The estrogen-induced inhibition of the response to vascular injury and the preventive effect of estrogen against atherosclerosis occur over a period of hours or days after estrogen treatment and are dependent on changes in gene expression in the vascular tissues; these actions are sometimes referred to as "genomic."

There are two estrogen receptors, estrogen receptor α and estrogen receptor β, both of which are members of the superfamily of steroid hormone receptors. (Walter P., et al., Proc Nad Acad Sci USA 1985;82:7889-93; Kuiper G.G.J.M., et al., Proc Nad Acad Sci USA 1996;93:5925-30) Estrogen receptors α and β have considerable homology and, like all steroid hormone receptors, are transcription factors that alter gene expression when they are activated. (Walter P., et al., Proc Nad Acad Sci USA 1985;82:7889-93; Kuiper G.G.J.M., et al., Proc Nad Acad Sci USA 1996;93:5925-30; Shibata H., et al., Recent Prog Horm Res 1997;52:141-65; Evans R.M., Science 1988;240:889-95; Brown M., Hematol Oncol Clin North Am 1994;8:101-12). Blood vessels are complex structures, with walls containing smooth-muscle cells and an endothelial cell lining. Vascular endothelial and smooth muscle cells bind estrogen with high affinity (Mendelsohn M.E., et al., Curr Opin Cardiol 1994;9:619-26; Farhat M.Y., et al., FASEB J 1996;10:615-24) and estrogen receptor α has been identified in both types of vascular cells in women and men, (Karas R.H., et al., Circulation 1994;89:1943-50; Losordo D.W., et al., Circulation 1994;89:1501-10; Venkov C.D., et al., Circulation 1996;94:727-33; Kim-Schulze S., et al., Circulation 1996;94:1402-7; Caulin-Glaser T., et al., J Clin Invest 1996;98:36-42) as well as in myocardial cells (Grohe C., et al., FEBS Lett 1997;416:107-12).

Estrogen receptor α activates specific target genes in vascular smooth-muscle and endothelial cells (Karas R.H., et al., Circulation 1994;89:1943-50, Venkov C.D., et al., Circulation 1996;94:727-33; Kim-Schulze S., et al., Circulation 1996;94:1402-7; Caulin-Glaser T., et al., J Clin Invest 1996;98:36-42; Koike H., et al., J Vasc Surg 1996;23:477-82). Estrogen receptor β is structurally and functionally distinct from estrogen receptor α. Functional estrogen receptor β is also present in myocardial cells, in which it regulates the expression of nitric oxide synthases.

In premenopausal women, 17β-estradiol produced by the ovaries is the chief circulating estrogen. Serum estradiol concentrations are low in preadolescent girls and increase at menarche. In women, they range from about 100 pg per milliliter (367 pmol per liter) in the follicular phase to about 600 pg per milliliter (2200 pmol per liter) at the time of ovulation. They may rise to nearly 20,000 pg per milliliter (70,000 pmol per liter) during pregnancy. After menopause, serum estradiol concentrations fall to values similar to or lower than those in men of similar age (5 to 20 pg per milliliter [18 to 74 pmol per liter]) (Yen, S.S.C. and Jaffe, R.B. eds., Reproductive Endocrinology: Physiology, Pathophysiology and Clinical Management, 3rd ed. Philadelphia: W.B. Saunders, 1991).

While estrogenic effects can provide vascular benefits and prevent and reverse vaginal atrophy in postmenopausal women. The administration of estrogen alone can have deleterious effects. For example, breast cancer is a hormone-dependent disease. Women without functioning ovaries who never receive estrogen replacement do not develop breast cancer. The female-to-male ratio for the disease is about 150 to 1. A host of findings indicate that hormones play a critical role as promoters of the disease. For most epithelial malignancies, a log-log plot of incidence versus age shows a straight-line increase with every year of life. A similar plot for breast cancer shows the same straight-line increase, but with a decrease in slope beginning at the age of menopause. The three dates in a woman's life that have a major impact on breast cancer incidence are age of menarche, age at first full-term pregnancy, and age of menopause. Women who experience menarche at age 16 have only 50 to 60 percent of the lifetime breast cancer risk of women who experience menarche at age 12. Similarly, menopause occurring 10 years before the median age (52 years), whether natural or surgically induced, reduces lifetime breast cancer risk by about 35 percent. Compared with nulliparous women, women who have a first full-term pregnancy by age 18 have 30 to 40 percent the risk of breast cancer. Thus, length of menstrual life-particularly the fraction occurring before the first full-term pregnancy-is a substantial component of the total risk of breast cancer. This factor can account for 70 to 80 percent of the variation in breast cancer frequency in different countries.

International variation has provided some of the most important clues on hormonal carcinogenesis. A woman living to age 80 in North America has 1 chance in 9 of developing invasive breast cancer. Asian women have one-fifth to one-tenth the risk of breast cancer of women in North America or Western Europe. Asian women have substantially lower concentrations of estrogens and progesterone. These differences cannot be explained on a genetic basis, because Asian women living in a Western environment have a risk identical to that of their Western counterparts. These women also differ markedly in height and weight from Asian women in Asia; height and weight are critical regulators of age of menarche and have substantial effects on plasma concentrations of estrogens. (Lippman, M.E., *Breast Cancer,* Chapter 91, in Harrison's Principles of Internal Medicine, 14th ed., 1998). Thus despite the beneficial effects which estrogens play in maintaining health, the administration of estrogens may also cause adverse effects on a subject's health such as an increased risk of breast cancer.

Menopause occurs naturally at an average age of 50 to 51 years in the USA. As ovaries age, response to pituitary gonadotropins (follicle-stimulating hormone [FSH] and luteinizing hormone [LH]) decreases, initially resulting in shorter follicular phases (thus, shorter menstrual cycles), fewer ovulations, decreased progesterone production, and more irregularity in cycles. Eventually, the follicle fails to respond and does not produce estrogen. The transitional phase, during which a woman passes out of the reproductive stage, begins before menopause. It is termed the climacteric or perimenopause, although many persons refer to it as menopause.

Premature menopause refers to ovarian failure of unknown cause that occurs before age 40. It may be associated with smoking, living at high altitude, or poor nutritional status. Artificial menopause may result from oophorectomy, chemotherapy, radiation of the pelvis, or any process that impairs ovarian blood supply.

The compositions and methods of the present invention act to treat female sexual dysfunction. These effects are accomplished by the compositions and methods of the invention with a substantial reduction of the concomitant liability of adverse effects associated with estrogen administration.

For the treatment of female subject sexual dysfunction, the compositions of the present invention can be administered either singly or in combination with agents that elevate cyclic guanosine 3',5'-monophosphate (cGMP). Agents that elevate cGMP levels are well known and can work through any of several mechanisms. Agents which selectively inhibit an enzyme predominantly involved in cGMP breakdown, for example a cGMP-dependent phosphodiesterase constitute one example.

In particular, cyclic guanosine 3',5'-monophosphate phosphodiesterase (cGMP PDE) inhibitors are widely known as cardiovascular agents for the treatment of conditions such as angina, hypertension, and congestive heart failure. More recently cGMP PDE inhibitors capable of inhibiting type V phosphodiesterase (cGMP PDEᵥ) have been found to be effective for the treatment of male erectile dysfunction, importantly by oral administration. See, for example, PCT/EP94/01580, published as WO 94/28902 which designates, *inter alia,* the United States.

### BRIEF DESCRIPTION OF THE DRAWING

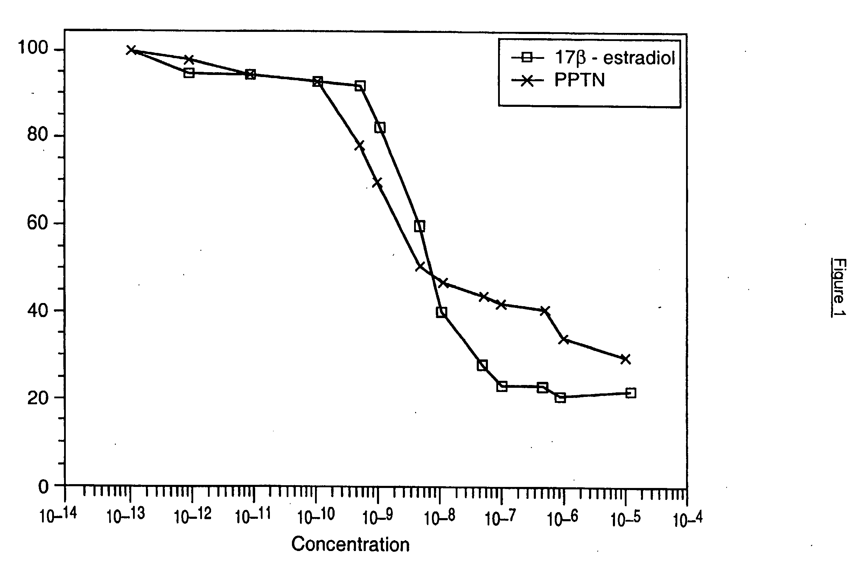

Figure 1 is a log-linear competition binding plot of PPTN and 17β-estradiol to human estrogen receptor. The X-axis represents percentage of radiolabeled estrogen bound to receptor. The Y-axis represents molar concentration of added ligand. Values are mean ± SEM.

### SUMMARY OF THE INVENTION

This invention relates to pharmaceutical compositions useful for treating female sexual dysfunction. The compositions are comprised of an estrogen agonist / antagonist and, optionally, a cGMP elevator and a pharmaceutically acceptable carrier, vehicle or diluent.

A second aspect of the invention relates to methods of treating female sexual dysfunction including hypoactive sexual desire disorder, sexual arousal disorder, dyspareunia and vaginismus. The methods comprise the administration of an effective amount of an estrogen agonist / antagonist and, optionally, the co-administration of a cyclic guanosine 3',5'-monophosphate elevator to a female subject and, preferably, a postmenopausal female subject.

A third aspect of the invention is that the estrogen agonists / antagonists and methods for treating female sexual dysfunction are effective while substantially reducing the concomitant liability of adverse effects associated with estrogen administration.

As a fourth aspect, the present invention provides for the use of estrogen agonists / antagonists and, optionally, cGMP elevators for the manufacture of a medicament to treat female sexual dysfunction, preferably in a postmenopausal female subject. These indications are also treated by the medicament while substantially reducing the concomitant liability of adverse effects associated with estrogen administration.

### DETAILED DESCRIPTION OF THE INVENTION

The present invention relates to compositions, methods and kits for treating female sexual dysfunction. Unless otherwise specified, the following terms have the meanings as defined below:

As used herein, "limit", "treat" and "treatment" are interchangeable terms as are "limiting" and "treating" and, as used herein, include preventative (e.g., prophylactic) and palliative treatment or the act of providing preventative or palliative treatment.

"Adverse effects associated with estrogen" include breast tenderness, bloating, headache, increased blood clotting and menstrual bleeding in women. Unopposed estrogen therapy increases the risk of endometrial carcinoma. Women on long-term estrogen therapy may have an increased risk that is not reversed by concurrent progestin (N Engl J Med 1995;332:1589).

The term "postmenopausal women" is defined to include not only women of advanced age who have passed through menopause, but also women who have been hysterectomized or for some other reason have suppressed estrogen production, such as those who have undergone long-term administration of corticosteroids, suffer from Cushions' syndrome or have gonadal dysgenesis.

"Breast cancer" is defined as a malignant proliferation of epithelial cells lining the ducts or lobules of the breast.

"Co-administration" of a combination of a estrogen agonist / antagonist and a cGMP elevator means that these components can be administered together as a composition or as part of the same, unitary dosage form. "Co-administration" also includes administering an estrogen agonist / antagonist and a cGMP elevator separately but as part of the same therapeutic treatment program or regimen. The components need not necessarily be administered at essentially the same time, although they can if so desired. Thus "co-administration" includes, for example, administering a estrogen agonist / antagonist and a cGMP elevator as separate dosages or dosage forms, but at the same time. "Co-administration" also includes separate administration at different times and in any order. For example, where appropriate a patient may take one or more component(s) of the treatment in the morning and the one or more of the other component(s) at night.

An "estrogen agonist / antagonist" is a compound that affects some of the same receptors that estrogen does, but not all, and in some instances, it antagonizes or blocks estrogen. It is also known as a "selective estrogen receptor modulator" (SERM). Estrogen agonists / antagonists may also be referred to as antiestrogens although they have some estrogenic activity at some estrogen receptors. Estrogen agonists / antagonists are therefore not what are commonly referred to as "pure antiestrogens". Antiestrogens that can also act as agonists are referred to as Type I antiestrogens. Type I antiestrogens activate the estrogen receptor to bind tightly in the nucleus for a prolonged time but with impaired receptor replenishment (Clark, et al., Steroids 1973;22:707, Capony et al., Mol Cell Endocrinol, 1975;3:233).

An estrogen agonist / antagonist and, optionally, a cGMP elevator when administered or co-administered either as part of the same pharmaceutical composition or as separate pharmaceutical compositions is/are effective in treating female sexual dysfunction. By treating female sexual dysfunction, the compositions and methods of the invention are suitable for treating a variety of conditions. These conditions encompass arousal, pain and orgasmic disorders such as: female sexual arousal disorder; hypoactive sexual desire disorder, sexual anhedonia; dyspareunia; and vaginismus. The estrogen agonists / antagonists and cyclic guanosine 3',5'-monophosphate elevators of the invention may be administered systemically or locally. For systemic use, the compounds herein are formulated for parenteral (e.g., intravenous, subcutaneous, intramuscular, intraperitoneal, intranasal or transdermal) or enteral (e.g., oral or rectal) delivery according to conventional methods. Intravenous administration can be by a series of injections or by continuous infusion over an extended period. Administration by injection or other routes of discretely spaced administration can be performed at intervals ranging from weekly to once to three times daily.

Preferred estrogen agonists / antagonists of the present invention include the compounds described in US 5,552,412. Those compounds are described by formula (I) given below: 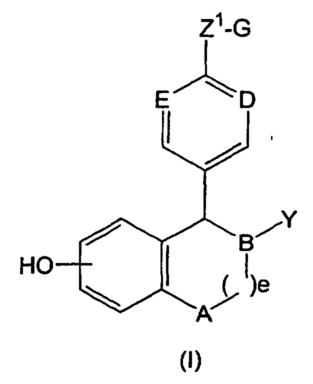 wherein:
A is selected from CH₂ and NR;
B, D and E are independently selected from CH and N;
Y is
   (a) phenyl, optionally substituted with 1-3 substituents independently selected from R⁴;
   (b) naphthyl, optionally substituted with 1-3 substituents independently selected from R⁴;
   (c) C₃-C₈ cycloalkyl, optionally substituted with 1-2 substituents independently selected from R⁴;
   (d) C₃-C₈ cycloalkenyl, optionally substituted with 1-2 substituents independently selected from R⁴;
   (e) a five membered heterocycle containing up to two heteroatoms selected from the group consisting of -O-, -NR²- and -S(O)ₙ-, optionally substituted with 1-3 substituents independently selected from R⁴;
   (f) a six membered heterocycle containing up to two heteroatoms selected from the group consisting of -O-, -NR²- and -S(O)ₙ- optionally substituted with 1-3 substituents independently selected from R⁴; or
   (g) a bicyclic ring system consisting of a five or six membered heterocyclic ring fused to a phenyl ring, said heterocydic ring containing up to two heteroatoms selected from the group consisting of -O-, -NR²- and -S(O)ₙ-, optionally substituted with 1-3 substituents independently selected from R⁴;
Z¹ is
   (a) -(CH₂)ₚW(CH₂)_{q}-;
   (b) -O(CH₂)ₚCR⁵R⁶-;
   (c) -O(CH₂)ₚW(CH₂)_{q}-;
   (d) -OCHR²CHR³-; or
   (e) -SCHR²CHR³-;
G is
   (a) -NR⁷R⁸;
   (b) 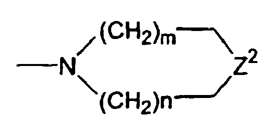 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
   (c) a bicyclic amine containing five to twelve carbon atoms, either bridged or fused and optionally substituted with 1-3 substituents independently selected from R⁴; or
Z¹ and G in combination may be 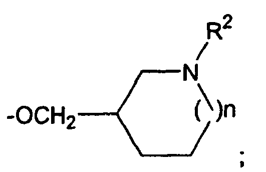
W is
   (a) -CH₂-;
   (b) -CH=CH-;
   (c) -O-;
   (d) -NR²-;
   (e) -S(O)ₙ-;
   (f) 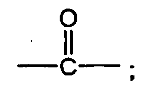
   (g) -CR²(OH)-;
   (h) -CONR²-;
   (i) -NR²CO-;
   (j) 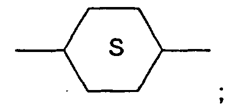 or
   (k) -C≡C-;
R is hydrogen or C₁-C₆ alkyl;
R² and R³ are independently
   (a) hydrogen; or
   (b) C₁-C₄ alkyl;
R⁴ is
   (a) hydrogen;
   (b) halogen;
   (c) C₁-C₆ alkyl;
   (d) C₁-C₄ alkoxy;
   (e) C₁-C₄ acyloxy;
   (f) C₁-C₄ alkylthio;
   (g) C₁-C₄ alkylsulfinyl;
   (h) C₁-C₄ alkylsulfonyl;
   (i) hydroxy (C₁-C₄)alkyl;
   (j) aryl (C₁-C₄)alkyl;
   (k) -CO₂H;
   (l) -CN;
   (m) -CONHOR;
   (n) -SO₂NHR;
   (o) -NH₂;
   (p) C₁-C₄ alkylamino;
   (q) C₁-C₄ dialkylamino;
   (r) -NHSO₂R;
   (s) -NO₂;
   (t) -aryl; or
   (u) -OH;
R⁵ and R⁶ are independently C₁-C₈ alkyl or together form a C₃-C₁₀ carbocyclic ring;
R⁷ and R⁸ are independently
   (a) phenyl;
   (b) a C₃-C₁₀ carbocyclic ring, saturated or unsaturated;
   (c) a C₃-C₁₀ heterocyclic ring containing up to two heteroatoms, selected from -O-, -N- and -S-;
   (d) H;
   (e) C₁-C₆ alkyl; or
   (f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
e is 0, 1 or 2;
m is 1, 2 or 3;
n is 0, 1 or 2;
p is 0, 1, 2 or 3;
q is 0, 1, 2 or 3;
and optical and geometric isomers thereof; and nontoxic pharmacologically acceptable acid addition salts, N-oxides, esters or quaternary ammonium salts thereof.

By halo is meant chloro, bromo, iodo, or fluoro or by halogen is meant chlorine, bromine, iodine or fluorine.

By alkyl is meant straight chain or branched chain saturated hydrocarbon. Exemplary of such alkyl groups (assuming the designated length encompasses the particular example) are methyl, ethyl, propyl, isopropyl, butyl, sec-butyl, tertiary butyl, pentyl, isopentyl, hexyl and isohexyl.

By alkoxy is meant straight chain or branched chain saturated alkyl bonded through an oxy. Exemplary of such alkoxy groups (assuming the designated length encompasses the particular example) are methoxy, ethoxy, propoxy, isopropoxy, butoxy, isobutoxy, tertiary butoxy, pentoxy, isopentoxy, hexoxy and isohexoxy.

The parenthetical negative or positive sign used herein in the nomenclature denotes the direction plane polarized light is rotated by the particular stereoisomer.

Additional preferred compounds of the invention are disclosed in U.S. Patent No.5,552,412 and are described by formula (IA): 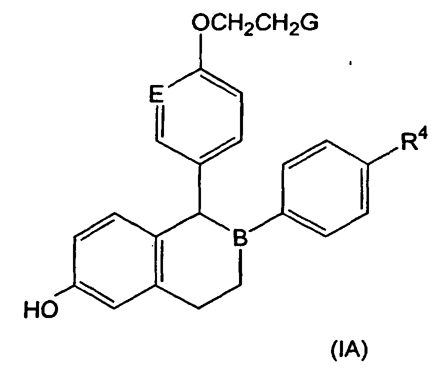 wherein G is 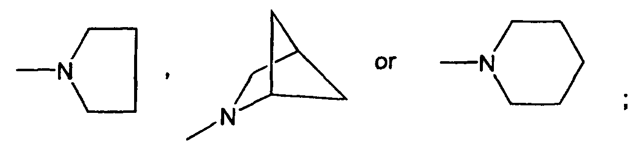 R⁴ is H, OH, F, or Cl; and B and E are independently selected from CH and N.

Especially preferred compounds for the compositions and methods of the invention are:
cis-6-(4-fluoro-phenyl)-5-[4-(2-piperidin-1-yl-ethoxy)-phenyl]-5,6,7,8-tetrahydro-naphthalene-2-ol;
(-)-cis-6-phenyl-5-[4-(2-pyrrolidin-1-yl-ethoxy)-phenyl]-5,6,7,8-tetrahydronaphthalene-2-ol;
cis-6-phenyl-5-[4-(2-pyrrolidin-1-yl-ethoxy)-phenyl]-5,6,7,8-tetrahydronaphthalene-2-ol;
cis-1-[6'-pyrrolidinoethoxy-3'-pyridyl]-2-phenyl-6-hydroxy-1,2,3,4-tetrahydronaphthalene;
1-(4'-pyrrolidinoethoxyphenyl)-2-(4"-fluorophenyl)-6-hydroxy-1,2,3,4-tetrahydroisoquinoline;
cis-6-(4-hydroxyphenyl)-5-[4-(2-piperidin-1-yl-ethoxy)-phenyl]-5,6,7,8-tetrahydro-naphthalene-2-ol;
1-(4'-pyrrolidinoethoxyphenyl)-2-phenyl-6-hydroxy-1,2,3,4-tetrahydroisoquinoline and pharmaceutically acceptable salts thereof. An especially preferred salt of (-)-cis-6-phenyl-5-[4-(2-pyrrolidin-1-yl-ethoxy)-phenyl]-5,6,7,8-tetrahydro-naphthalene-2-ol is the tartrate salt.

The pharmaceutically acceptable acid addition salts of the estrogen agonists / antagonists of this invention may be formed of the compound itself, or of any of its esters, and include the pharmaceutically acceptable salts which are often used in pharmaceutical chemistry. For example, salts may be formed with inorganic or organic acids such as hydrochloric acid, hydrobromic acid, hydroiodic acid, sulfonic acids including such agents as naphthalenesulfonic, methanesulfonic and toluenesulfonic acids, sulfuric acid, nitric acid, phosphoric acid, tartaric acid, pyrosulfuric acid, metaphosphoric acid, succinic acid, formic acid, phthalic acid, lactic acid and the like, most preferable with hydrochloric acid, citric acid, benzoic acid, maleic acid, acetic acid and propionic acid.

The estrogen agonists / antagonists of this invention, as discussed above, can be administered in the form of acid addition salts. The salts are conveniently formed by reacting a compound, if basic, with a suitable acid, such as have been described above. The salts are quickly formed in high yields at moderate temperatures, and often are prepared by merely isolating the compound from a suitable acidic wash as the final step of the synthesis. The salt-forming acid is dissolved in an appropriate organic solvent, or aqueous organic solvent, such as an alkanol, ketone or ester. On the other hand, if the compound of this invention is desired in the free base form, it is isolated from a basic final wash step, according to the usual practice. A preferred technique for preparing hydrochlorides is to dissolve the free base in a suitable solvent and dry the solution thoroughly, as over molecular sieves, before bubbling hydrogen chloride gas through it. A preferred salt of (-)-cis-6-phenyl-5-[4-(2-pyrrolidin-1-yl-ethoxy)-phenyl]-5,6,7,8-tetrahydronaphthalene-2-ol is the D-(-)-tartrate salt. It will also be recognized that it is possible to administer amorphous forms of the estrogen agonists / antagonists.

For the treatment of female sexual dysfunction, cGMP elevator agents may be coadministered with the estrogen agonist / antagonists of the present invention either separately or in a single composition.

For the treatment of male subject sexual dysfunction, cGMP elevator agents may be coadministered with the estrogen agonist / antagonists of the present invention either separately or in a single composition.

Preferred as the cGMP elevator are cGMP PDE inhibitors. cGMP PDE inhibitors which are selective for cGMP PDEs rather than cyclic adenosine 3',5'-monophosphate phosphodiesterases (cAMP PDEs) and/or which are selective inhibitors of the cGMP PDEᵥ isoenzyme are particularly preferred. Such particularly preferred cGMP PDE inhibitors are disclosed in US patents 5,250,534; 5,346,901; 5,272,147, and in the international patent application published as WO 94/28902 designating, *inter alia*, the U.S.

Preferred cGMP PDEᵥ (also called PDE5) inhibitors include compounds of formula (VII): 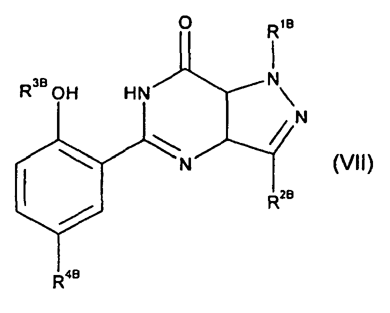 wherein:
R^{1B} is H; C₁-C₃ alkyl; C₁-C₃ perfluoroalkyl; or C₃-C₅ cycloalkyl;
R^{2B} is H; C₁-C₆ alkyl optionally substituted with C₃-C₆ cycloalkyl; C₁-C₃ perfluoroalkyl; or C₃-C₆ cycloalkyl;
R^{3B} is C₁-C₆ alkyl optionally substituted with C₃-C₆ cycloalkyl; C₁-C₆ perfluoroalkyl; C₃-C₅ cycloalkyl; C₃-C₆ alkenyl; or C₃-C₆ alkynyl;
R^{4B} is C₁-C₄ alkyl optionally substituted with OH, NR^{5B}R^{6B}, CN, CONR^{5B}R^{6B} or CO₂R^{7B}; C₂-C₄ alkenyl optionally substituted with CN, CONR^{5B}R^{6B} or CO₂R^{7B}; C₂-C₄ alkanoyl optionally substituted with NR^{5B}R^{6B}; (hydroxy)C₂-C₄ alkyl optionally substituted with NR^{5B}R^{6B}; (C₂-C₃ alkoxy)C₁-C₂ alkyl optionally substituted with OH or NR^{5B}R^{6B}; CONR^{5B}R⁶B CO₂R^{7B}; halo; NR^{5B}R^{6B}; NHSO₂NR^{5B}R^{6B}; NHSO₂R^{8B}; SO₂NR^{9B}R^{10B} or phenyl, pyridyl, pyrimidinyl, imidazolyl, oxazolyl, thiazolyl, thienyl or triazolyl any of which is optionally substituted with methyl;
R^{5B} and R^{6B} are each independently H or C₁-C₄ alkyl, or together with the nitrogen atom to which they are attached form a pyrrolidinyl, piperidino, morpholino, 4-N(R^{11B})-piperazinyl or imidazolyl group wherein said group is optionally substituted with methyl or OH;
R^{7B} is H or C₁-C₄ alkyl;
R^{8B} is C₁-C₃ alkyl optionally substituted with NR^{5B}R^{6B};
R^{9B} and R^{10B} together with the nitrogen atom to which they are attached form a pyrrolidinyl, piperidino, morpholino or 4-N(R^{12B})-piperazinyl group wherein said group is optionally substituted with C₁-C₄ alkyl, C₁-C₃ alkoxy, NR^{13B}R^{14B} or CONR^{13B}R^{14B};
R^{11B} is H; C₁-C₃ alkyl optionally substituted with phenyl; (hydroxy)C₂-C₃ alkyl; or C₁-C₄ alkanoyl;
R^{12B} is H; C₁-C₆ alkyl; (C₁-C₃ alkoxy)C₂-C₆ alkyl; (hydroxy)C₂-C₆ alkyl; (R^{13B}R^{14B}N)C₂-C₆ alkyl; (R^{13B}R^{14B}NOC)C₁-C₆ alkyl; CONR^{13B}R^{14B}; CSNR^{13B}R^{14B}; or C(NH)NR^{13B}R^{14B}; and
R^{13B} and R^{14B} are each independently H; C₁-C₄ alkyl; (C₁-C₃ alkoxy)C₂-C₄ alkyl; or (hydroxy)C₂-C₄ alkyl;
or a pharmaceutically acceptable salt thereof;
or a pharmaceutically acceptable composition containing either entity.

Preferred cGMP PDEᵥ inhibitors include sildenafil (preferably the citrate salt) {1-[[3-(6,7-dihydro-1-methyl-7-oxo-3-propyl-1H-pyrazolo[4,3-d]pyrimidin-5-yl)-4-ethoxy-phenyl]sulfonyl]-4-methylpiperazine}, which has the structure of formula (VIII): 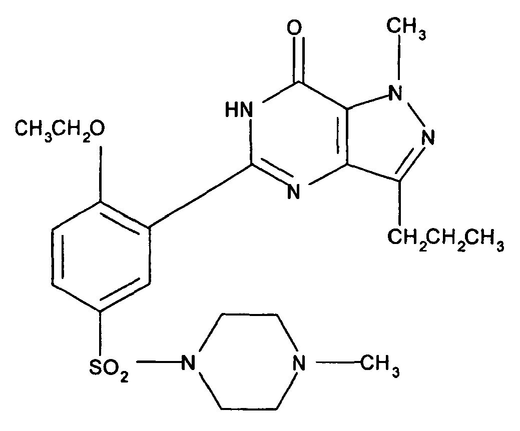 and pharmaceutically acceptable salts thereof, the compound having the structure of formula (IX): 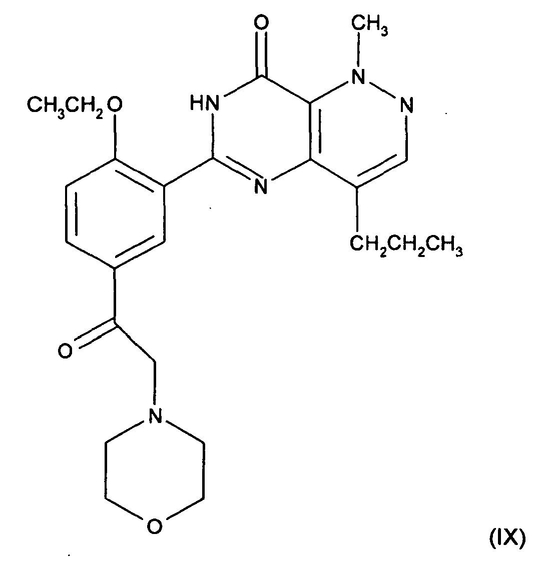 and pharmaceutically acceptable salts thereof, and the compound, 3-ethyl-5-{5-[(4-ethylpiperazino) sulphonyl]-2-(2-methoxyethoxy)pyrid-3-yl}-2-(2-pyridylmethyl)-6,7-dihydro-2H-pyrazolo[4,3-d]pyrimidin-7-one of formula (X) below:

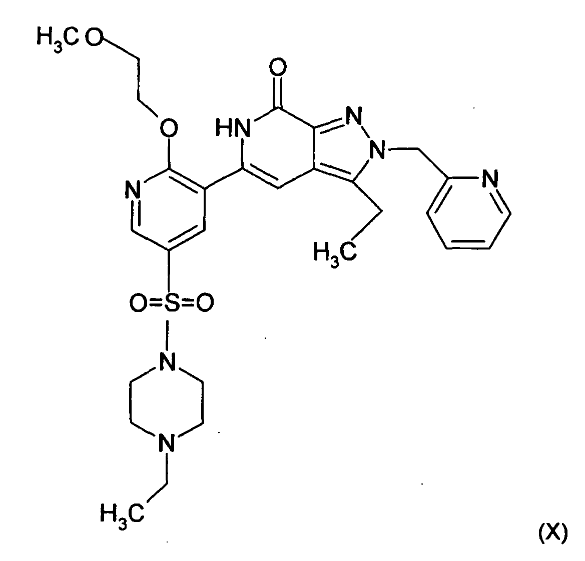

The compound of formula (IX) is disclosed, for example, in US Patents 5,272,147 and 5,426,107.

Also preferred as cGMP PDEᵥ inhibitors are compounds disclosed in PCT/EP95/00183, published as WO 95/19978 and which designates, *inter alia,* the United States, said compounds having the formula (XI): 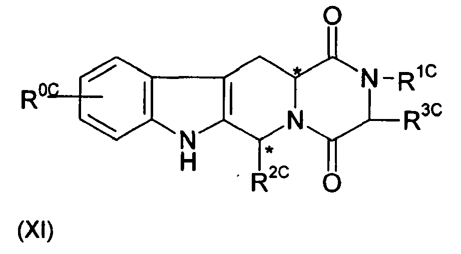 and salts and solvates thereof, in which:
R^{0C} represents hydrogen, halogen or C₁-C₆alkyl,;
R^{1C} represents hydrogen, C₁-C₆alkyl, C₂-C₆alkenyl, C₂-C₆alkynyl, haloC₁-C₆alkyl, C₃-C₈cycloalkyl, C₃-C₈cycloalkylC₁-C₃alkyl, arylC₁-C₃alkyl or heteroarylC₁-C₃alkyl;
R^{2C} represents an optionally substituted monocyclic aromatic ring selected from benzene, thiophene, furan and pyridine or an optionally substituted bicyclic ring 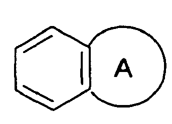 attached to the rest of the molecule via one of the benzene ring carbon atoms and wherein the fused ring A is a 5- or 6-membered ring which may be saturated or partially or fully unsaturated and comprises carbon atoms and optionally one or two heteroatoms selected from oxygen, sulphur and nitrogen; and R^{3C} represents hydrogen or C₁-C₃alkyl, or R^{1C} and R^{3C} together represent a 3- or 4-membered alkyl or alkenyl ring.

A preferred subset of compounds having formula XIa (also disclosed in WO 95/19978) includes compounds of the formula: 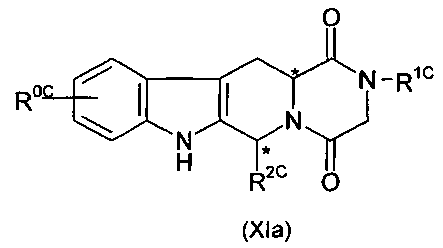 and salts and solvates thereof, in which:
R^{0c} represents hydrogen, halogen or C₁-C₆alkyl;
R^{1c} represents hydrogen, C₁-C₆alkyl, haloC₁-C₆alkyl, C₃-C₈cycloalkyl,
C₃-C₈cycloalkyl-C₁-C₃alkyl, arylC₁-C₃alkyl or heteroarylC₁-C₃alkyl; and
R^{2c} represents an optionally substituted monocyclic aromatic ring selected from benzene, thiophene, furan and pyridine or an optionally substituted bicyclic ring 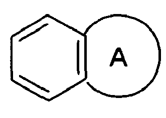 attached to the rest of the molecule via one of the benzene ring carbon atoms and wherein the fused ring A is a 5- or 6-membered ring which may be saturated or partially or fully unsaturated and comprises carbon atoms and optionally one or two heteroatoms selected from oxygen, sulphur and nitrogen.

Suitable cGMP PDE5 inhibitors for the use according to the present invention include: the pyrazolo [4,3-d]pyrimidin-7-ones disclosed in EP-A-0463756; the pyrazolo [4,3-d]pyrimidin-7-ones disclosed in EP-A-0526004; the pyrazolo [4,3-d]pyrimidin-7-ones disclosed in published international patent application WO 93/06104; the isomeric pyrazolo [3,4-d]pyrimidin-4-ones disclosed in published international patent application WO 93/07149; the quinazolin-4-ones disclosed in published international patent application WO 93/12095; the pyrido [3,2-d]pyrimidin-4-ones disclosed in published international patent application WO 94/05661; the purin-6-ones disclosed in published international patent application WO 94/00453; the pyrazolo [4,3-d]pyrimidin-7-ones disclosed in published international patent application WO 98/49166; the pyrazolo [4,3-d]pyrimidin-7-ones disclosed in published international patent application WO 99/54333; the pyrazolo [4,3-d]pyrimidin-4-ones disclosed in EP-A-0995751; the pyrazolo [4,3-d]pyrimidin-7-ones disclosed in published international patent application WO 00/24745; the pyrazolo [4,3-d]pyrimidin-4-ones disclosed in EP-A-0995750; the compounds disclosed in published international application WO95/19978; the compounds disclosed in published international application WO 99/24433 and the compounds disclosed in published international application WO 93/07124.

Preferred type V phosphodiesterase inhibitors for the use according to the present invention include: 5-[2-ethoxy-5-(4-methyl-1-piperazinylsulphonyl)phenyl]-1-methyl-3-n-propyl-1,6-dihydro-7H-pyrazolo[4,3-d]pyrimidin-7-one (sildenafil) also known as 1-[[3-(6,7-dihydro-1-methyl-7-oxo-3-propyl-1H-pyrazolo[4,3-d]pyrimidin-5-yl)-4-ethoxyphenyl]sulphonyl]-4-methylpiperazine (see EP-A-0463756);
5-(2-ethoxy-5-morpholinoacetylphenyl)-1-methyl-3-n-propyl-1,6-dihydro-7H-pyrazolo[4,3-d]pyrimidin-7-one (see EP-A-0526004);
3-ethyl-5-[5-(4-ethylpiperazin-1-ylsulphonyl)-2-n-propoxyphenyl]-2-(pyridin-2-yl)methyl-2,6-dihydro-7H-pyrazolo[4,3-d]pyrimidin-7-one (see WO98/49166);
3-ethyl-5-[5-(4-ethylpiperazin-1-ylsulphonyl)-2-(2-methoxyethoxy)pyridin-3-yl]-2-(pyridin-2-yl)methyl-2,6-dihydro-7H-pyrazolo[4,3-d]pyrimidin-7-one (see WO99/54333);
(+)-3-ethyl-5-[5-(4-ethylpiperazin-1-ylsulphonyl)-2-(2-methoxy-1(R)-methylethoxy)pyridin-3-yl]-2-methyl-2,6-dihydro-7H-pyrazolo[4,3-d]pyrimidin-7-one, also known as 3-ethyl-5-{5-[4-ethylpiperazin-1-ylsulphonyl]-2-([(1R)-2-methoxy-1-methylethyl]oxy)pyridin-3-yl}-2-methyl-2,6-dihydro-7H-pyrazolo[4,3-d] pyrimidin-7-one (see WO99/54333);
5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one, also known as 1-{6-ethoxy-5-[3-ethyl-6,7-dihydro-2-(2-methoxyethyl)-7-oxo-2H-pyrazolo[4,3-d]pyrimidin-5-yl]-3-pyridylsulphonyl}-4-ethylpiperazine (see Example 1 hereinafter);
5-[2-*iso*-Butoxy-5-(4-ethylpiperazin-1-ylsulphonyl)pyridin-3-yl]-3-ethyl-2-(1-methylpiperidin-4-yl)-2,6-dihydro-7H-pyrazolo[4,3-d]pyrimidin-7-one (see Example 2 hereinafter);
5-[2-Ethoxy-5-(4-ethylpiperazin-1-ylsulphonyl)pyridin-3-yl]-3-ethyl-2-phenyl-2,6-dihydro-7H-pyrazolo[4,3-d]pyrimidin-7-one (see Example 3 hereinafter);
5-(5-Acetyl-2-propoxy-3-pyridinyl)-3-ethyl-2-(1-isopropyl-3-azetidinyl)-2,6-dihydro-7H-pyrazolo[4,3-d]pyrimidin-7-one (see Example 4 hereinafter);
5-(5-Acetyl-2-butoxy-3-pyridinyl)-3-ethyl-2-(1-ethyl-3-azetidinyl)-2,6-dihydro-7H-pyrazolo[4,3-*d*]pyrimidin-7-one (see Example 5 hereinafter);
(6R,12aR)-2,3,6,7,12,12a-hexahydro-2-methyl-6-(3,4-methylenedioxyphenyl) - pyrazino[2',1':6,1]pyrido[3,4-b]indole-1,4-dione (IC-351), i.e. the compound of examples 78 and 95 of published international application WO95/19978 as well as the compound of examples 1, 3, 7 and 8;
2-[2-ethoxy-5-(4-ethyl-piperazin-1-yl-1-sulphonyl)-phenyl]-5-methyl-7-propyl-3H-imidazo[5,1-f][1,2,4]triazin-4-one (vardenafil) also known as 1-[[3-(3,4-dihydro-5-methyl-4-oxo-7-propylimidazo[5,1-f]-as-triazin-2-yl)-4-ethoxyphenyl]sulphonyl]-4-ethylpiperazine, i.e., the compound of examples 20, 19, 337 and 336 of published international application WO99/24433; and the compound of example 11 of published international application WO93/07124 (EISAI); and compounds 3 and 14 from Rotella D P, J. Med. Chem., 2000, 43, 1257.

Still other type cGMP PDE5 inhibitors useful in conjunction with the present invention include:4-bromo-5-(pyridylmethylamino)-6-[3-(4-chlorophenyl)-propoxy]-3(2H)pyridazinone; 1-[4-[(1,3-benzodioxol-5-ylmethyl)amiono]-6-chloro-2-quinazolinyl]-4-piperidine-carboxylic acid, monosodium salt; (+)-cis-5,6a,7,9,9,9a-hexahydro-2-[4-(trifluoromethyl)-phenylmethyl-5-methyl-cyclopent-4,5]imidazo[2,1-b]purin-4(3H)one; furazlocillin; cis-2-hexyl-5-methyl-3,4,5,6a,7,8,9,9a-octahydrocyclopent[4,5]-imidazo[2,1-b]purin-4-one; 3-acetyl-1-(2-chlorobenzyl)-2-propylindole-6- carboxylate; 3-acetyl-1-(2-chlorobenzyl)-2-propylindole-6-carboxylate; 4-bromo-5-(3-pyridylmethylamino)-6-(3-(4-chlorophenyl) propoxy)-3-(2H)pyridazinone; I-methyl-5(5-morpholinoacetyl-2-n-propoxyphenyl)-3-n-propyl-1,6-dihydro- 7H-pyrazolo(4,3-d)pyrimidin-7-one; 1-[4-[(1,3-benzodioxol-5-ylmethyl)arnino]-6-chloro-2- quinazolinyl]-4-piperidinecarboxylic acid, monosodium salt; Pharmaprojects No. 4516 (Glaxo Wellcome); Pharmaprojects No. 5051 (Bayer); Pharmaprojects No. 5064 (Kyowa Hakko; see WO 96/26940); Pharmaprojects No. 5069 (Schering Plough); GF-196960 (Glaxo Wellcome); E-8010 and E-4010 (Eisai); Bay-38-3045 & 38-9456 (Bayer) and Sch-51866.

The suitability of any particular cGMP PDE5 inhibitor can be readily determined by evaluation of its potency and selectivity using literature methods followed by evaluation of its toxicity, absorption, metabolism, pharmacokinetics, etc in accordance with standard pharmaceutical practice.

Preferably, the cGMP PDE5 inhibitors have an IC₅₀ at less than 100 nanomolar, more preferably, at less than 50 nanomolar, more preferably still at less than 10 nanomolar.

IC₅₀ values for the cGMP PDE5 inhibitors may be determined using established literature methodology, for example as described in EP0463756-B1 and EP0526004-A1.

Preferably the cGMP PDE5 inhibitors used are selective for the PDE5 enzyme. Preferably they are selective over PDE3, more preferably over PDE3 and PDE4. Preferably, the cGMP PDE5 inhibitors have a selectivity ratio greater than 100 more preferably greater than 300, over PDE3 and more preferably over PDE3 and PDE4.

Selectivity ratios may readily be determined by the skilled person. IC50 values for the PDE3 and PDE4 enzyme may be determined using established literature methodology, see S A Ballard *et al.,* Journal of Urology, 1998, vol. 159, pages 2164-2171.

### cGMP Example 1

2-(Methoxyethyl)-5-[2-ethoxy-5-(4-ethylpiperazin-1-ylsulphonyl)pyridin-3-yl]-3-ethyl-2,6-dihydro-7H-pyrazolo[4,3-d]pyrimidin-7-one

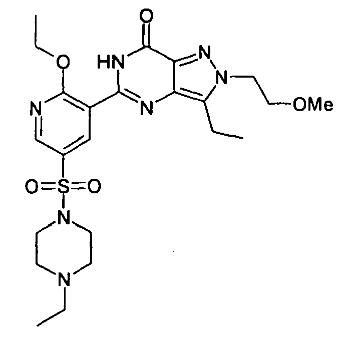

A mixture of the product from stage i) below (0.75mmol), potassium bis(trimethylsilyl)amide (298mg, 1.50mmol) and ethyl acetate (73 microlitres, 0.75mmol) in ethanol (10ml) was heated at 120°C in a sealed vessel for 12 hours. The cooled mixture was partitioned between ethyl acetate and aqueous sodium bicarbonate solution, and the layers separated. The organic phase was dried (MgSO₄), and evaporated under reduced pressure. The crude product was purified by column chromatography on silica gel using dichloromethane:methanol (98:2) as eluant to afford the title compound, 164mg; Found : C, 53.18; H, 6.48; N, 18.14; C₂₃H₃₃N₇O₅S;0.20C₂H₅CO₂CH₃ requires C, 53.21; H, 6.49; N, 18.25%; δ (CDCl₃) : 1.04 (3H, t), 1.40 (3H, t), 1.58 (3H, t), 2.41 (2H, q), 2.57 (4H, m), 3.08 (2H, q), 3.14 (4H, m), 3.30 (3H, s), 3.92 (2H, t), 4.46 (2H, t), 4.75 (2H, q), 8.62 (1H, d), 9.04 (1H, d), 10.61 (1H, s); LRMS : m/z 520 (M+1)⁺; mp 161-162°C.

### Preparation of Starting Materials

### a) Pyridine-2-amino-5-sulphonic acid

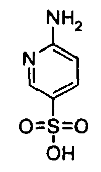

2-Aminopyridine (80g, 0.85mol) was added portionwise over 30 minutes to oleum (320g) and the resulting solution heated at 140°C for 4 hours. On cooling, the reaction was poured onto ice (200g) and the mixture stirred in an ice/salt bath for a further 2 hours. The resulting suspension was filtered, the solid washed with ice water (200ml) and cold IMS (200ml) and dried under suction to afford the title compound as a solid, 111.3g; LRMS : m/z 175 (M+1)⁺

### b) Pyridine-2-amino-3-bromo-5-sulphonic acid

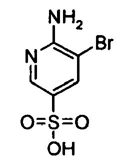

Bromine (99g, 0.62mol) was added dropwise over an hour, to a hot solution of the product from stage a) (108g, 0.62mol) in water (600ml) so as to maintain a steady reflux. Once the addition was complete the reaction was cooled and the resulting mixture filtered. The solid was washed with water and dried under suction to afford the title compound, 53.4g; δ (DMSOd₆, 300MHz): 8.08 (1H, s), 8.14 (1H, s); LRMS : m/z 253 (M)⁺.

### c) Pyridine-3-bromo-2-chloro-5-sulphonyl chloride

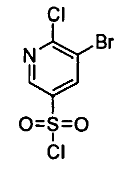

A solution of sodium nitrite (7.6g, 110.0mmol) in water (30ml) was added dropwise to an ice-cooled solution of the product from stage b) (25.3g, 100.0mmol) in aqueous hydrochloric acid (115m), 20%), so as to maintain the temperature below 6°C. The reaction was stirred for 30 minutes at 0°C and for a further hour at room temperature. The reaction mixture was evaporated under reduced pressure and the residue dried under vacuum at 70°C for 72 hours. A mixture of this solid, phosphorus pentachloride (30.0g, 144mmol) and phosphorus oxychloride (1ml, 10.8mmol) was heated at 125°C for 3 hours, and then cooled. The reaction mixture was poured onto ice (100g) and the resulting solid filtered, and washed with water. The product was dissolved in dichloromethane, dried (MgSO₄), and evaporated under reduced pressure to afford the title compound as a yellow solid, 26.58g; δ (CDCl₃, 300MHz) : 8.46 (1H, s), 8.92 (1H, s).

### d) 3-Bromo-2-chloro-5-(4-ethylpiperazin-1-ylsulphonyl)pyridine

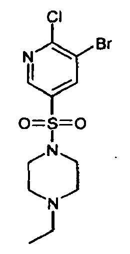

A solution of 1-ethylpiperazine (11.3ml, 89.0mmol) and triethylamine (12.5ml, 89.0mmol) in dichloromethane (150ml) was added dropwise to an ice-cooled solution of the product from stage c) (23.0g, 79.0mmol) in dichloromethane (150ml) and the reaction stirred at 0°C for an hour. The reaction mixture was concentrated under reduced pressure and the residual brown oil was purified by column chromatography on silica gel, using an elution gradient of dichloromethane:methanol (99:1 to 97:3) to afford the title compound as an orange solid, 14.5g; δ (CDCl₃, 300MHz) : 1.05 (3H, t), 2.42 (2H, q), 2.55 (4H, m), 3.12 (4H, m), 8.24 (1H, s), 8.67 (1H, s).

### e) 3-Bromo-2-ethoxy-5-(4-ethylpiperazin-1-ylsulphonyl)pyridine

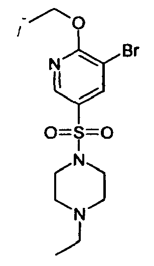

A mixture of the product from stage d) (6.60g, 17.9mmol) and sodium ethoxide (6.09g, 89.55mmol) in ethanol (100ml) was heated under reflux for 18 hours, then cooled. The reaction mixture was concentrated under reduced pressure, the residue partitioned between water (100ml) and ethyl acetate (100ml), and the layers separated. The aqueous phase was extracted with ethyl acetate (2x100ml), the combined organic solutions dried (MgSO₄) and evaporated under reduced pressure to afford the title compound as a brown solid, 6.41g; Found : C, 41.27; H, 5.33; N, 11.11. C₁₃H₂₀BrN₃O₃S requires C, 41.35; H, 5.28; N, 10.99%; δ (CDCl₃, 300MHz) : 1.06 (3H, t), 1.48 (3H, t), 2.42 (2H, q), 2.56 (4H, m), 3.09 (4H, m), 4.54 (2H, q), 8.10 (1H, s), 8.46 (1H, s); LRMS : m/z 378, 380 (M+1)⁺.

### f) Pyridine 2-ethoxy-5-(4-ethylpiperazin-1-ylsulphonyl)-3-carboxylic acid ethyl ester

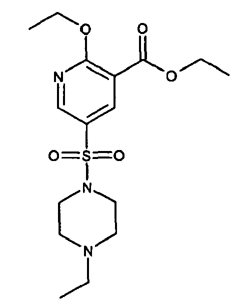

A mixture of the product from stage e) (6.40g, 16.92mmol), triethylamine (12ml, 86.1mmol), and palladium (0) tris(triphenylphosphine) in ethanol (60ml) was heated at 100°C and 200 psi, under a carbon monoxide atmosphere, for 18 hours, then cooled. The reaction mixture was evaporated under reduced pressure and the residue purified by column chromatography on silica gel, using an elution gradient of dichloromethane:methanol (100:0 to 97:3) to afford the title compound as an orange oil, 6.2g; δ (CDCl₃, 300MHz) : 1.02 (3H, t), 1.39 (3H, t), 1.45 (3H, t), 2.40 (2H, q), 2.54 (4H, m), 3.08 (4H, m), 4.38 (2H, q), 4.55 (2H, q), 8.37 (1H, s), 8.62 (1H, s); LRMS : m/z 372 (M+1)⁺

### g) Pyridine 2-ethoxy-5-(4-ethylpiperazin-1-ylsulphonyl)-3-carboxylic acid

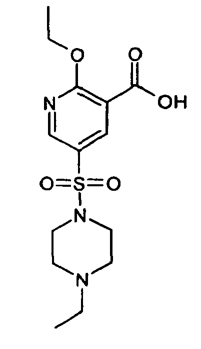

A mixture of the product from stage f) (4.96g, 13.35mmol) and aqueous sodium hydroxide solution (25ml, 2N, 50.0mmol) in ethanol (25ml) was stirred at room temperature for 2 hours. The reaction mixture was concentrated under reduced pressure to half it's volume, washed with ether and acidified to pH 5 using 4N hydrochloric acid. The aqueous solution was extracted with dichloromethane (3x30ml), the combined organic extracts dried (MgSO₄) and evaporated under reduced pressure to afford the title compound as a tan coloured solid, 4.02g; δ (DMSOd₆, 300MHz): 1.18 (3H, t), 1.37 (3H, t), 3.08 (2H, q), 3.17-3:35 (8H, m), 4.52 (2H, q), 8.30 (1H, s), 8.70 (1H, s).

### h) 4-[2-Ethoxy-5-(4-ethylpiperazin-1-ylsulphonyl)pyridin-3-ylcarboxamido]-1H-3-ethylpyrazole-5-carboxamide

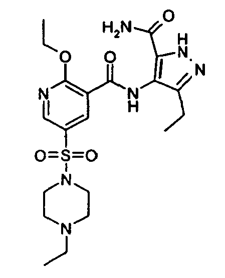

A solution of 4-amino-3-ethyl-1H-pyrazole-5-carboxamide (WO 9849166, preparation 8) (9.2g, 59.8mmol) in N,N-dimethylformamide (60ml) was added to a solution of the product from stage g) (21.7g, 62.9mmol), 1-hydroxybenzotriazole hydrate (10.1g, 66.0mmol) and triethylamine (13.15ml, 94.3mmol) in dichloromethane (240ml). 1-(3-Dimethylaminopropyl)-3-ethylcarbodiimide hydrochloride (13.26g, 69.2mmol) was added and the reaction stirred at room temperature for 6 hours. The dichloromethane was removed under reduced pressure, the remaining solution poured into ethyl acetate (400ml), and this mixture washed with aqueous sodium bicarbonate solution (400ml). The resulting crystalline precipitate was filtered, washed with ethyl acetate and dried under vacuum, to afford the title compound, as a white powder, 22g; δ (CDCl₃+1 drop DMSOd₆) 0.96 (3H, t), 1.18 (3H, t), 1.50 (3H, t), 2.25-2.56 (6H, m), 2.84 (2H, q), 3.00 (4H, m), 4.70 (2H, q), 5.60 (1H, br s), 6.78 (1H, br s), 8.56 (1H, d), 8.76 (1H, d), 10.59 (1H, s), 12.10-12.30 (1H, s); LRMS: m/z 480 (M+1)⁺.

### i) 2-Methoxyethyl-4-[2-ethoxy-5-(4-ethylpiperazin-1-ylsulphonyl)pyridin-3-ylcarboxamido]-3-ethylpyrazole-5-carboxamide

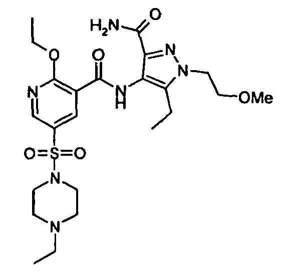

1-Bromo-2-methoxyethane (1.72mmol) was added to a solution of the product from stage h) (750mg, 1.56mmol) and caesium carbonate (1.12g, 3.44mmol) in N,N-dimethylformamide (15ml) and the reaction stirred at 60°C for 18 hours. The cooled mixture was partitioned between water and ethyl acetate, and the layers separated. The organic layer was dried (MgSO₄), concentrated under reduced pressure and azeotroped with toluene to give a solid. This product was recrystallised from ether, to afford the title compound as a white solid.

### cGMP Example 2

### 5-[2-iso-Butoxy-5-(4-ethylpiperazin-1-ylsulphonyl)pyridin-3-yl]-3-ethyl-2-(1-methylpiperidin-4-yl)-2,6-dihydro-7H-pyrazolo[4,3-d]pyrimidin-7-one

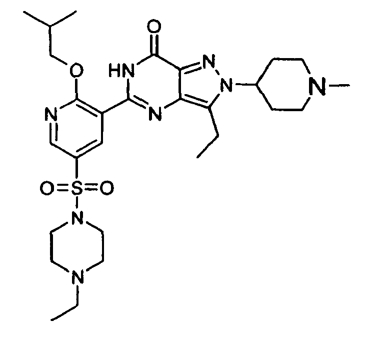

A mixture of the product from stage b) below (90mg, 0.156mmol), potassium bis(trimethylsilyl)amide (156mg, 0.78mmol) and ethyl acetate (14mg, 0.156mmol) in iso-propanol (12ml) was stirred at 130°C for 6 hours in a sealed vessel. The cooled reaction mixture was poured into saturated aqueous sodium bicarbonate solution (60ml), and extracted with ethyl acetate (60ml). The combined organic extracts were dried (MgSO₄), and evaporated under reduced pressure to give a gum. The crude product was purified by column chromatography on silica gel using dichloromethane: methanol: 0.88 ammonia (92.6:6.6:0.6) to afford the title compound as a beige foam, 36 mg; δ (CDCl₃) 1.01 (3H, t), 1.12 (6H, d), 1.39 (3H, t), 1.94 (2H, m), 2.15 (2H, m), 2.22-2.44 (6H, m), 2.55 (6H, m), 3.02 (4H, m), 3.14 (4H, m), 4.22 (1H, m), 4.43 (2H, d), 8.60 (1H, d), 9.00 (1H, d), 10.54 (1H, s).

### Preparation of Starting Materials

### a) 2-(1-tert-Butoxycarbonylpiperidin-4-yl)-4-[2-ethoxy-5-(4-ethylpiperazin-1-ylsulphonyl)pyridin-3-ylcarboxamido]-3-ethylpyrazole-5-carboxamide

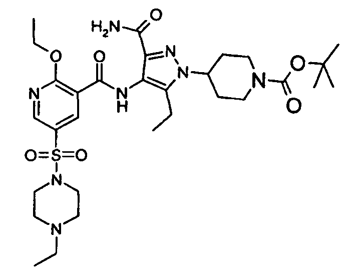

Sodium hydride (64mg, 60% dispersion in mineral oil, 1.6mmol) was added to a solution of the product from Example 1, stage h) (1.46mmol) in tetrahydrofuran (10ml), and the solution stirred for 10 minutes. *tert*-Butyl 4-[(methylsulphonyl)oxy]-1-piperidinecarboxylate (WO 9319059) (1.60mmol) was added and the reaction stirred at 60°C for 3 days. The cooled mixture was partitioned between ethyl acetate and aqueous sodium bicarbonate solution, and the phases separated. The aqueous layer was extracted with ethyl acetate, the combined organic solutions dried (MgSO₄) and evaporated under reduced pressure. The residue was purified by column chromatography on silica gel using dichloromethane:methanol (98:2) as eluant to afford the title compound as a white foam, 310 mg; δ (CDCl₃) 1.02 (3H, t), 1.23 (3H, t), 1.49 (9H, s), 1.57 (3H, m), 1.93 (2H, m), 2.16 (2H, m), 2.40 (2H, q), 2.54 (4H, m), 2.82-2.97 (4H, m), 3.10 (4H, m), 4.30 (3H, m), 4.79 (2H, q), 5.23 (1H, s), 6.65 (1H, s), 8.63 (1H, d), 8.82 (1H, d), 10.57 (1H, s).

### b) 4-[2-Ethoxy-5-(4-ethylpiperazin-1-ylsulphonyl)pyridin-3-ylcarboxamidol-3-ethyl-2-(1-methylpiperidin-4-yl)pyrazole-5-carboxamide

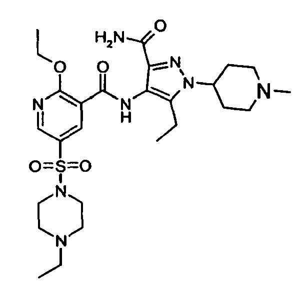

Trifluoroacetic acid (1.5ml) was added to a solution of the product from stage a) above (320mg, 0.48mmol) in dichloromethane (2ml) and the solution stirred at room temperature for 2 ½ hours. The reaction mixture was evaporated under reduced pressure and the residue triturated well with ether and dried under vacuum, to provide a white solid. Formaldehyde (217 microlitres, 37% aqueous, 2.90mmol) was added to a solution of the intermediate amine in dichloromethane (8ml), and the solution stirred vigorously for 30 minutes. Acetic acid (88 microlitres, 1.69mmol) was added, the solution stirred for a further 30 minutes, then sodium triacetoxyborohydride (169mg, 0.80mmol) was added and the reaction stirred at room temperature for 16 hours. The reaction mixture was poured into aqueous sodium bicarbonate solution, and extracted with ethyl acetate. The combined organic extracts were dried (MgSO₄) and evaporated under reduced pressure. The residue was purified by column chromatography on silica gel using dichloromethane:methanol:0.88 ammonia (91.75:7.5:0.75) as eluant to afford the title compound, 70mg; δ (CDCl₃) 1.02 (3H, t), 1.22 (3H, t), 1.58 (3H, t), 1.92 (2H, m), 2.14 (2H, m), 2.25-2.45 (7H, m), 2.54 (4H, m), 2.91 (2H, q), 2.99-3.16 (6H, m), 4.08 (1H, m), 4.78 (2H, q), 5.11 (1H, br s), 6.65 (1H, br s), 8.63 (1H, d), 8.83 (1H, d), 10.53 (1H, s).

### cGMP Example 3

### 5-[2-Ethoxy-5-(4-ethylpiperazin-1-ylsulphonyl)pyridin-3-yl]-3-ethyl-2-phenyl-2,6-dihydro-7H-pyrazolo[4,3-d]pyrimidin-7-one

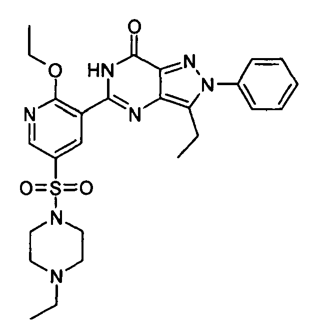

Pyridine (0.1ml, 1.08mmol) was added to a mixture of the product from stage a) below (250mg, 0.54mmol), copper (II) acetate monohydrate (145mg, 0.72mmol), benzeneboronic acid (132mg, 1.08mmol) and 4A molecular sieves (392mg) in dichloromethane (5ml), and the reaction stirred at room temperature for 4 days. The reaction mixture was filtered and the filtrate evaporated under reduced pressure. The crude product was purified by column chromatography on silica gel using dichloromethane:methanol:0.88 ammonia (97:3:0.5) as eluant, and triturated with ether:hexane. The resulting solid was filtered and recrystallised from iso-propanol:dichloromethane to give the title compound as a solid, 200mg, δ (CDCl₃) 1.02 (3H, t), 1.47 (3H, t), 1.60 (3H, t), 2.42 (2H, q), 2.58 (4H, m), 3.10 (2H, q), 3.17 (4H, m), 4.76 (2H, q), 7.40 (1H, m), 7.51 (2H, m), 7.80 (2H, d), 8.67 (1H, d), 9.16 (1H, s), 10.90 (1H, s); LRMS : m/z 538 (M+1)⁺.

### Preparation of Starting Materials

### a) 5-[2-Ethoxy-5-(4-ethylpiperazin-1-ylsulphonyl)pyridin-3-yl]-3-ethyl-2,6-dihydro-7H-pyrazolo[4,3-d]pyrimidin-7-one

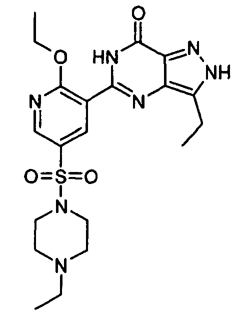

Potassium bis(trimethylsilyl)amide (8.28g, 41.6mmol) was added to a solution of the product from Example 1, stage h) (10.0g, 20.8mmol) and ethyl acetate (2ml, 20mmol) in ethanol (160ml), and the reaction mixture heated at 120°C for 12 hours in a sealed vessel. The cooled mixture was evaporated under reduced pressure and the residue was purified by column chromatography on silica gel using dichloromethane:methanol:0.88 ammonia (95:5:0.5) as eluant, to give the title compound, 3.75g; δ (CDCl₃) 1.03 (3H, t), 1.42 (3H, t), 1.60 (3H, t), 2.42 (2H, q), 2.58 (4H, m), 3.02 (2H, q), 3.16 (4H, m), 4.78 (2H, q), 8.66 (1H, d), 9.08 (1H, d), 11.00 (1H, s) 11.05-11.20 (1H, br s), LRMS : m/z 462 (M+1)⁺.

### cGMP Example 4

### 5-(5-Acetyl-2-propoxy-3-pyridinyl)-3-ethyl-2-(1-isopropyl-3-azetidinyl)-2,6-dihydro-7H-pyrazolo[4,3-d]pyrimidin-7-one

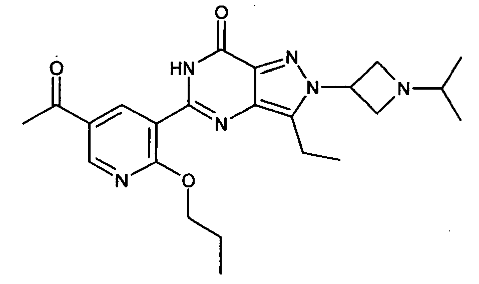

The product from stage h) below (0.23 mmol) was dissolved in dichloromethane (10 ml) and acetone (0.01 ml) was added. After 30 min stirring sodium triacetoxyborohydride (0.51 mmol) was added and stirring continued for 14 h. Further acetone (0.01 ml) and sodium triacetoxyborohydride (0.51 mmol) were added and stirring continued for a further 4.5 h. Starting material still remained so further acetone (0.01 ml) and sodium triacetoxyborohydride (0.51 mmol) were added and stirring continued for a further 18 h. The reaction mixture was diluted with dichloromethane, washed with sodium bicarbonate solution then brine, dried (MgSO₄) and concentrated. Purification by flash column chromatography (elution with 94:6:0.6 dichloromethane/methanol/0.88 ammonia) gave the product as a solid, M.p. 162.8-163.6°C; 1H NMR (400MHz, MeOD): δ = 1.00 (app. d, 9H), 1.30 (t, 3H), 1.84 (app. q, 2H), 2.60 (s, 3H), 2.62-2.72 (m, 1H), 3.00-3.10 (q, 2H), 3.75 (t, 2H), 3.90 (t, 2H), 4.50 (t, 2H), 5.25 (t, 1H), 8.70 (s, 1H), 8.90 (s, 1H); LRMS (TSP - positive ion) 439 (MH⁺); Anal. Found C, 61.92; H, 6.84; N, 18.70 Calcd for C₂₃H₃₀O₃N₆.0.1CH₂Cl₂: C, 62.07; H, 6.81; N, 18.80.

### Preparation of Starting Materials

### a) 2-Propoxy-5-iodonicotinic acid

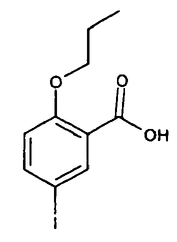

*N*-Iodosuccinamide (18.22 g, 0.08 mol), trifluoroacetic acid (100 ml) and trifluoroacetic anhydride (25 ml) were added to 2-propoxynicotinic acid (0.054 mol). The mixture was refluxed for 2.5 h, cooled and the solvents evaporated. The residue was extracted from water with ethyl acetate and the organics washed with water (twice) and brine (twice), dried (MgSO₄) and concentrated. The red residue was redissolved in ethyl acetate washed with sodium thiosulfate solution (twice), water (twice), brine (twice), redried (MgSO₄) and concentrated to give the desired product as a solid; ¹H NMR (300 MHz, CDCl₃): δ = 1.05 (t, 3H), 1.85-2.0 (m, 2H), 4.5 (t, 2H), 8.5 (s, 1H), 8.6 (s, 1H); Analysis: found C, 35.16; H, 3.19; N, 4.46. Calcd for C₉H₁₀INO₃: C, 35.19; H, 3.28; N, 4.56%.

### b) N-[3-(Aminocarbonyl)-5-ethyl-1H-pyrazol-4-yl]-5-iodo-2-propoxy-nicotinamide

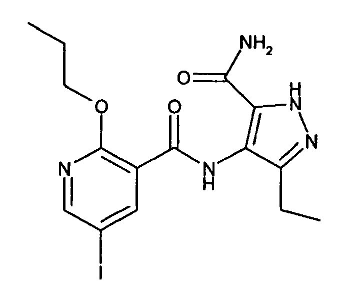

Oxalyl chloride (15.9 mmol) was added to a stirred solution of the product from stage a) (3.98 mmol) in dichloromethane (20 ml) and 3 drops N,N dimethylformamide added. After 2.5 h the solvent was evaporated and the residue azeotroped 3 times with dichloromethane. The residue was resuspended in dichloromethane (4 ml) and added to a stirred mixture 4-amino-3-ethyl-1*H*-pyrazole-5-carboxamide (prepared as described in WO 98/49166) (3.58 mmol) and triethylamine (7.97 mmol) in dichloromethane (10 ml). After 1 h the solvent was evaporated and the residue partitioned between ethyl acetate and water. The organic phase was separated and washed with 2N HCl (twice), sodium bicarbonate solution (twice) and brine before being dried (MgSO₄) and concentrated. The product was triturated with ether and filtered to give the title product as a solid. The mother liquor was concentrated and purified by flash column chromatography (elution with 80% ethyl acetate : hexane) to give further product; ¹H NMR (300 MHz, d₄-MeOH): δ = 1.0 (t, 3H), 1.25 (t, 3H), 1.85-2.0 (m, 2H), 2.8 (q, 2H), 4.5 (t, 2H), 8.5 (s, 1H), 8.6 (s, 1H); LRMS (TSP) 444 (MH⁺).

### c) tert-Butyl 3-iodo-1-azetidinecarboxylate

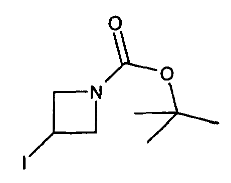

A mixture of *tert*-butyl 3-[(methylsulfonyl)oxy]-l-azetidinecarboxylate (prepared as described in *Synlett* 1998, 379; 5.0 g, 19.9 mmol), and potassium iodide (16.5 g, 99.4 mmol) in *N,N*-dimethylformamide (25 ml), was heated at 100°C for 42 h. The cooled mixture was partitioned between water and ethyl acetate, and the layers separated. The organic phase was dried over MgSO₄, concentrated under reduced pressure and the residue azeotroped with xylene. The crude product was purified by flash column chromatography (dichloromethane_as eluant) to give the title compound, 3.26 g; ¹H NMR (300 MHz, CDCl₃) δ = 1.43 (s, 9H), 4.28 (m, 2H), 4.46 (m, 1H), 4.62 (m, 2H); LRMS (TSP) 284 (MH)⁺

### d) tert-Butyl 3-(3-(aminocarbonyl)-5-ethyl-4-{[(5-iodo-2-propoxy-3-pyridinyl)carbonyl]amino}-1H-pyrazol-1-yl)-1-azetidinecarboxylate

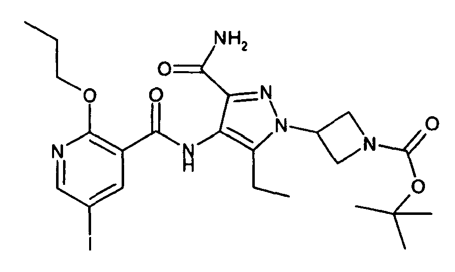

Cesium carbonate (3.59 mmol) was added to a stirred solution of the product from stage b) (1.79 mmol) and the product from stage c) (2.15 mmol) in *N,N-*dimethylformamide (10 ml) under a nitrogen atmosphere. The mixture was heated at 80°C for 24 h. The mixture was cooled and extracted from water with ethyl acetate. The organics were dried (MgSO₄) and concentrated to give a brown oil. Purification by flash column chromatography (gradient elution from 100% dichloromethane to 90% dichloromethane/MeOH) gave the title product; 1H NMR (400MHz, DMSO): δ = 0.95 (t, 3H), 1.05 (t, 3H), 1.40 (s, 9H), 1.78-1.88 (m, 2H), 2.68 (q, 2H), 4.22-4.35 (m, 4H), 4.40 (t, 2H), 5.33 (t, 1H), 7.35 (bs, 1H), 7.52 (bs, 1H), 8.40 (s, 1H), 8.55 (s, 1H), 10.10 (s, 1H); LRMS (TSP - positive ion) 373.2 (MH⁺ - BOC and I); Anal. Found C, 45.11; H, 5.07; N, 13.56 Calcd for C₂₃H₃₁O₅N₆I. 0.2 DCM: C, 45.28; H, 5.14; N, 13.66.

### e) tert-Butyl 3-[3-ethyl-5-(5-iodo-2-propoxy-3-pyridinyl)-7-oxo-6,7-dihydro-2H-pyrazolo[4,3-d]pyrimidin-2-yl]-1-azetidinecarboxylate

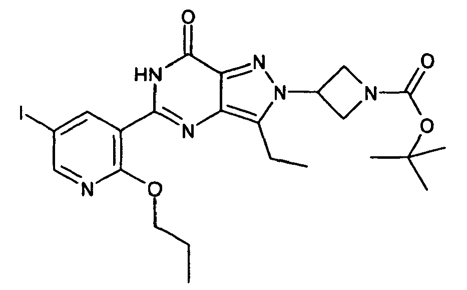

The product from stage d) (28.4 mmol) was dissolved in n-propanol (200 ml), ethyl acetate (6 ml) and potassium t-butoxide (28.4 mmol) were added and the resultant mixture heated to reflux for 6h. Additional potassium t-butoxide (14.2 mmol) was added and the mixture heated for a further 2h, after which the solvent was removed *in vacuo.* The residue was partioned between water (50 ml) and methylene chloride (100 ml) and the organic phase separated. The aqueous phase was extracted with dichloromethane (2 x 100 ml) and the combined organics dried over MgSO₄ and reduced to a solid. Purification by column chromatography (elution with ethyl acetate) gave the title compound; 1H NMR (400MHz, CDCl₃): δ = 1.05 (t, 3H), 1.30 (t, 3H), 1.43 (s, 9H), 1.87-1.96 (m, 2H), 3.00 (q, 2H), 4.34 (t, 2H), 4.49 (t, 2H), 4.60 (br s, 2H), 5.20 (t, 1H), 8.41 (d, 1H), 8.94 (s, 1H), 10.75 (br s, 1H); LRMS (TSP - positive ion) 598.1 (MNH₄⁺); Anal. Found C, 47.54; H, 5.02; N, 14.09 Calcd for C₂₃H₂₉O₄N₆l: C, 47.60; H, 5.04; N, 14.48.

### f) tert-Butyl 3-(3-ethyl-7-oxo-5-{2-propoxy-5-[(trimethylsilyl)ethynyl]-3-pyridinyl}-6,7-dihydro-2H-pyrrazolo[4,3-d]pyrimidin-2-yl)-1-azetidinecarboxylate

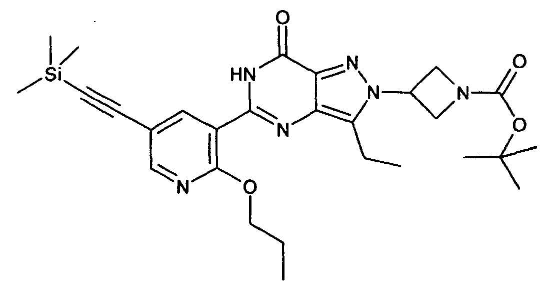

The product from stage e) (0.25 mmol) was suspended in triethylamine (2 ml) and trimethylsilylacetylene (0.39 mmol) and acetonitrile (2 ml to try and solubilise reactants). Pd(PPh₃)₂Cl₂ (0.006 mmol) and cuprous iodide (0.006 mmol) were added and the reaction mixture stirred. After 1 h a further portion of trimethylsilylacetylene (0.19 mmol) was added and stirring continued for 2 h. The solvent was evaporated and the residue partitioned between ethyl acetate and water. The organics were washed with brine, dried (MgSO₄) and concentrated. Purification by flash column chromatography (gradient elution from 100% dichloromethane to 99% dichloromethane/methanol) gave the title compound; 1H NMR (400MHz, MeOD): δ = 0.25 (s, 9H), 1.05 (t, 3H), 1.31 (t, 3H), 1.44 (s, 9H), 1.87-1.96 (m, 2H), 3.00 (q, 2H), 4.33 (t, 2H), 4.52 (t, 2H), 4.54-4.80 (m, 2H), 5.18-5.25 (m, 1H), 8.32 (d, 1H), 8.74 (d, 1H); LRMS (TSP - positive ion) 569 (MNH₄⁺), 452.0 (MH⁺); Anal. Found C, 60.82; H, 6.90; N, 15.15 Calcd for C₂₈H₃₈O₄N₆Si: C, 61.07; H, 6.95; N, 15.26.

### g) tert-Butyl 3-[3-ethyl-5-(5-ethynyl-2-propoxy-3-pyridinyl)-7-oxo-6,7-dihydro-2H-pyrazolo[4,3-d]pyrimidin-2-yl]-1-azetidinecarboxylate

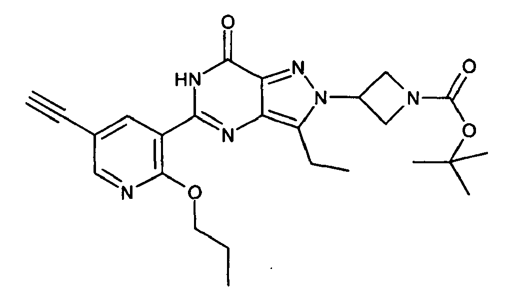

Potassium fluoride (0.38 mmol) was added to a stirred solution of the product of stage f) (0.19 mmol) in aqueous *N,N*-dimethylformamide (2 ml *N,N-*dimethylformamide /0.2 ml water) at 0°C. After 10 min the reaction was allowed to warm to room temperature and stirred for 2 h. The reaction mixture was diluted with ethyl acetate and washed with water, 1 N hydrochloric acid (3 times) and brine. The organic layer was dried (MgSO₄) and concentrated to give the title compound as a solid; 1H NMR (400MHz, CDCl₃): δ = 1.05 (t, 3H), 1.30 (t, 3H), 1.43 (s, 9H), 1.88-2.00 (m, 2H), 3.00 (q, 2H), 3.19 (s, 1H), 4.35 (app t, 2H), 4.52 (app t, 2H), 4.60-4.80 (br s, 2H), 5.22 (t, 1H), 8.39 (s, 1H), 8.80 (s, 1H), 10.75 (br s, 1H); LRMS (TSP - positive ion) 496 (MNH₄⁺).

### h) 5-(5-Acetyl-2-propoxy-3-pyridinyl)-2-(3-azetidinyl)-3-ethyl-2,6-dihydro-7H-pyrazolo[4,3-d]pyrimidin-7-one

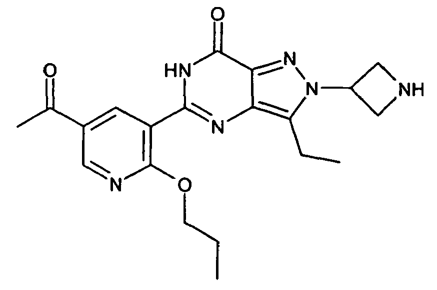

The product from stage g) (1.44 g, 3.0 mmol) in acetone (50 ml) and sulphuric acid (1N, 3 ml) was treated with mercuric sulphate (268 mg, 9.0 mmol) and heated to reflux for 6h. The reaction mixture was concentrated to ~20 ml *in vacuo,* poured into sodium bicarbonate (sat. aq., 20ml) and extracted into methylene chloride (6 x 20 ml). Combined organics were washed with brine (20 ml), dried over MgSO₄, and concentrated to a brown oil which was taken up in 40% trifluoroacetic acid in methylene chloride (50ml) and water (1 ml) and stirred for 1 h at room temperature. After evaporation *in vacuo,* the residue was purified by column chromatography (eluting with 95:5:1 methylene chloride:methanol:0.88 ammonia) to afford the title compound as a white hydroscopic foam (1.65 g); m.p. 128.5-1-30.0°C; 1H NMR (400MHz, MeOD): δ = 1.00 (t, 3H), 1.30 (t, 3H), 1.79-1.90 (m, 2H), 2.60 (s, 3H), 3.00-3.10 (q, 2H), 4.50 (t, 2H), 4.60-4.70 (m, 4H), 5.65-5.78 (m, 1H), 8.65 (s, 1H), 8.90 (s, 1H); LRMS (TSP - positive ion) 397 (MH⁺).

### cGMP Example 5

### 5-(5-Acetyl-2-butoxy-3-pyridinyl)-3-ethyl-2-(1-ethyl-3-azetidinyl)-2.6-dihydro-7H-pyrazolo[4,3-d]pyrimidin-7-one

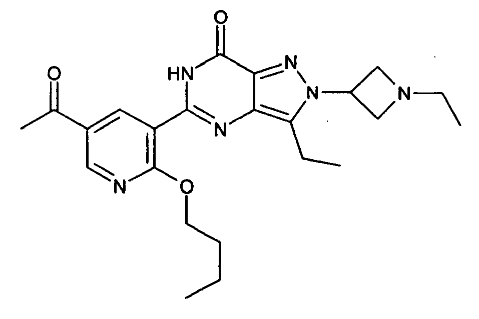

The starting material (120 mg, 0.28 mmol) and cesium carbonate (274 mg, 0.84 mmol) were dissolved in *n*-butanol (4 ml), and heated at 90°C under nitrogen with molecular sieves for 96h. The mixture was then partitioned between water (10 ml) and dichloromethane (10 ml). The organic layer was separated, and the aqueous layer extracted further with dichloromethane (3 x 15 ml). The combined organic layers were dried (MgSO₄), and concentrated *in vacuo.* The crude product was purified by flash column chromatography (95:5:0.5-90:10:1 ethyl acetate:methanol:0.88 NH₃ as eluents), to yield the title compound as a colourless glass (77 mg, 0.18 mmol); m.p. 91.6-93.7°C; 1H NMR (400MHz, CDCl₃): δ = 1.00-1.05 (m, 6H), 1.38 (t, 3H), 1.50-1.62 (m, 2H), 1.90-2.00 (m, 2H), 2.63 (s, 3H), 2.63-2.70 (m, 2H), 3.02 (q, 2H), 3.75 (t, 2H), 3.90 (t, 2H), 4.68 (t, 2H), 5.10-5.20 (m, 1H), 8.84 (s, 1H), 9.23 (s, 1H), 10.63 (br s, 1H); LRMS (TSP - positive ion) 439 (MH⁺); Anal. Found C, 60.73; H, 7.06; N, 18.03 Calcd for C₂₃H₃₀O₃N₆.0.2MeOH.0.1 DIPE: C, 60.88; H, 7.26; N, 17.90.

### Preparation of Starting Materials

### 5-(5-Acetyl-2-propoxy-3-pyridinyl)-3-ethyl-2-(1-ethyl-3-azetidinyl)-2,6-dihydro-7H-pyrazolo[4,3-d]pyrimidin-7-one

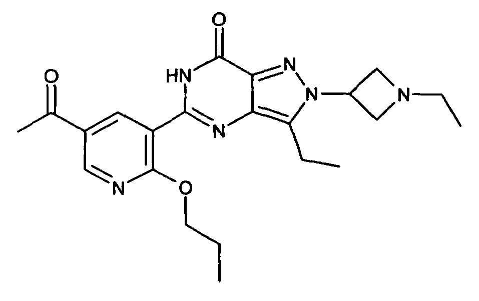

Sodium cyanoborohydride (92 mg, 1.47 mmol) was added to a stirred solution of the product from Example 4 stage h) (500 mg, 0.98 mmol) and sodium acetate (161 mg, 1.96 mmol) in methanol (10 ml) under nitrogen at room temperature. After 1h the mixture was poured into NaHCO₃ (sat. aq., 20 ml), and extracted with dichloromethane (3 x 15 ml). The combined organic layers were dried (MgSO₄) and concentrated *in vacuo.* The crude product was purified by flash column chromatography (95:5:0.5-80:20:1 ethyl acetate:methanol:0.88 NH₃ as eluent) to yield the title compound as a white solid (140 mg, 0.33 mmol); 1H NMR (400MHz, CDCl₃): δ = 0.97 (t, 3H), 1.03 (t, 3H), 1.30 (t, 3H), 2.82-2.97 (m, 2H), 2.58-2.65 (m, 5H), 2.98 (q, 2H), 3.68 (t, 2H), 3.85 (dd, 2H), 4.58 (dd, 2H), 5.05-5.17 (m, 1H), 8.79 (s, 1H), 9.18 (s, 1H), 10.62 (br s, 1H); LRMS (TSP - positive ion) 426 (MH⁺).

Oral daily dosages of the above cGMP elevators can range from about 1 mg to about 200 mg with a preferred range of from about 20 mg to about 100 mg. Dosage is *ad libitum* from about 15 minutes to about 4 hours prior to sexual activity. Dosages and timing of dosing can be adjusted for topical dosage forms such as creams or aerosols. cGMP elevators of the present invention include produgs, stereoisomers, hydrates, tautomers and salts of the described compounds. The cGMP elevators of the present invention may be formulated and administered as described for the estrogen agonists / antagonists above.

The cGMP PDE inhibitors useful in this invention as cGMP elevators may be chosen from among any of those already known to the art or subsequently discovered and/or hereafter developed. Suitable cGMP PDE inhibitors include those disclosed in any of the following US patents:
a 5-substituted pyrazolo[4,3-d]pyrimidine-7-one as disclosed in US 4,666,908;
a griseolic acid derivative as disclosed in any of US 4,634,706, 4,783,532, 5,498,819, 5,532,369, 5,556,975, and 6,616,600;
a 2-phenylpurinone derivative as disclosed in US 4,885,301;
a phenylpyridone derivative as disclosed in US 5,254,571;
a fused pyrimidine derivative as disclosed in US 5,047,404;
a condensed pyrimidine derivative as disclosed in US 5,075,310;
a pyrimidopyrimidine derivative as disclosed in US 5,162,316;
a purine compound as disclosed in US 5,073,559;
a quinazoline derivative as disclosed in US 5,147,875;
a phenylpyrimidone derivative as disclosed in US 5,118,686;
an imidazoquinoxalinone derivative or its aza analog as disclosed in US 5,055,465 and 5,166,344;
a phenylpyrimidone derivative as disclosed in US 5,290,933;
a 4-aminoquinazoline derivative as disclosed in US 5,436,233 or 5,439,895;
a 4,5-dihydro-4-oxo-pyrrolo[1,2-a]quinoxaline derivative as disclosed in US 5,405, 847;
a polycyclic guanine derivative as disclosed in US 5,393,755;
a nitogenous heterocyclic compound as disclosed in US 5,576,322;
a quinazoline derivative as disclosed in US 4,060,615;
a 6-heterocyclyl pyrazolo[3,4-d]pyrimidin-4-one as disclosed in US 5,294,612; and
a 4-aminoquinazoline derivative as disclosed in US 5,436,233;

Other disclosures of cGMP PDE inhibitors include the following:
European patent Application (EPA) publication no. 0428268;
European patent 0442204;
International patent application publication no. WO 94/19351;
Japanese patent application 5-222000;
European Journal of Pharmacology, 251, (1994), 1;
International patent application publication no. WO 94/22855;
a pyrazolopyrimidine derivative as disclosed in European patent application 0636626;
a 4-aminopyrimidine derivative as disclosed in European patent application 0640599;
an imidazoquinazoline derivative as disclosed in International patent application WO95/06648;
an anthranilic acid derivative as disclosed in International patent application WO95/18097;
a tetracyclic derivative as disclosed in International patent application WO95/19978;
an imidazoquinazoline derivative as disclosed in European patent application 0668280; and
a quinazoline compound as disclosed in European patent application 0669324.

The cGMP PDE inhibition of a compound can be determined by standard assays known to the art, for example as disclosed in US 5,250,534. Compounds which are selective inhibitors of cGMP PDE relative to cAMP PDE are preferred, and determination of such compounds is also taught in US 5,250,534. Particularly preferred are compounds which selectively inhibit the PDEᵥ isoenzyme, as disclosed in the aforementioned PCT/EP94/01580, published as WO 94/28902.

It will be recognized that certain of the above cGMP elevators contain either a free carboxylic acid or a free amine group as part of the chemical structure. Further, certain cGMP elevators within the scope of this invention contain lactone moieties, which exist in equilibrium with the free carboxylic acid form. These lactones can be maintained as carboxylates by preparing pharmaceutically acceptable salts of the lactone. Thus, this invention includes pharmaceutically acceptable salts of those carboxylic acids or amine groups. The expression "pharmaceutically acceptable salts" includes both pharmaceutically acceptable acid addition salts and pharmaceutically acceptable cationic salts. The expression "pharmaceutically-acceptable cationic salts" is intended to define but is not limited to such salts as the alkali metal salts, (e.g. sodium and potassium), alkaline earth metal salts (e.g., calcium and magnesium), aluminum salts, ammonium salts, and salts with organic amines such as benzathine (N,N'-dibenzylethylenediamine), choline, diethanolamine, ethylenediamine, meglumine (N-methylglucamine), benethamine (N-benzylphenethylamine), diethylamine, piperazine, tromethamine (2-amino-2-hydroxymethyl-1,3-propanediol) and procaine. The expression "pharmaceutically-acceptable acid addition salts" is intended to define but is not limited to such salts as the hydrochloride, hydrobromide, sulfate, hydrogen sulfate, phosphate, hydrogen phosphate, dihydrogenphosphate, acetate, succinate, citrate, methanesulfonate (mesylate) and p-toluenesulfonate (tosylate) salts. It will also be recognized that it is possible to administer amorphous forms of the cGMP elevators.

The pharmaceutically-acceptable cationic salts of cGMP elevators containing free carboxylic acids may be readily prepared by reacting the free acid form of the cGMP elevator with an appropriate base, usually one equivalent, in a co-solvent. Typical bases are sodium hydroxide, sodium methoxide, sodium ethoxide, sodium hydride, potassium methoxide, magnesium hydroxide, calcium hydroxide, benzathine, choline, diethanolamine, piperazine and tromethamine. The salt is isolated by concentration to dryness or by addition of a non-solvent. In many cases, salts are preferably prepared by mixing a solution of the acid with a solution of a different salt of the cation (e.g., sodium or potassium ethylhexanoate, magnesium oleate), employing a solvent (e.g., ethyl acetate) from which the desired cationic salt precipitates, or can be otherwise isolated by concentration and/or addition of a non-solvent.

The pharmaceutically acceptable acid addition salts of cGMP elevators containing free amine groups may be readily prepared by reacting the free base form of the cGMP elevator with the appropriate acid. When the salt is of a monobasic acid (e.g., the hydrochloride, the hydrobromide, the p-toluenesulfonate, the acetate), the hydrogen form of a dibasic acid (e.g., the hydrogen sulfate, the succinate) or the dihydrogen form of a tribasic acid (e.g., the dihydrogen phosphate, the citrate), at least one molar equivalent and usually a molar excess of the acid is employed. However when such salts as the sulfate, the hemisuccinate, the hydrogen phosphate or the phosphate are desired, the appropriate and exact chemical equivalents of acid will generally be used. The free base and the acid are usually combined in a co-solvent from which the desired salt precipitates, or can be otherwise isolated by concentration and/or addition of a non-solvent.

One of ordinary skill in the art will recognize that certain estrogen agonist / antagonists and cGMP elevators of this invention will contain one or more atoms which may be in a particular stereochemical, tautomeric, or geometric configuration, giving rise to stereoisomers, tautomers and configurational isomers. All such isomers and mixtures thereof are included in this invention. Hydrates and solvates of the compounds of this invention are also included.

The subject invention also includes isotopically-labeled estrogen agonists / antagonists and cGMP elevators, which are structurally identical to those disclosed above, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, sulfur, fluorine and chlorine, such as ²H, ³H, ¹³C, ¹⁴C, ¹⁵N, ¹⁸O, ¹⁷O, ³¹P, ³²P, ³⁵S, ¹⁸F and ³⁶Cl, respectively. Compounds of the present invention, prodrugs thereof, and pharmaceutically acceptable salts of said compounds and of said prodrugs which contain the aforementioned isotopes and/or other isotopes of other atoms are within the scope of this invention. Certain isotopically labeled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, may afford certain therapeutic advantages resulting from greater metabolic stability, for example increased *in vivo* half-life or reduced dosage requirements and, hence, may be preferred in some circumstances. Isotopically labeled compounds of this invention and prodrugs thereof can generally be prepared by carrying out known or referenced procedures and by substituting a readily available isotopically labeled reagent for a non-isotopically labeled reagent.

Pharmaceutical chemists will easily recognize that physiologically active compounds which have accessible hydroxy groups are frequently administered in the form of pharmaceutically acceptable esters. The literature concerning such compounds, such as estradiol, provides a great number of instances of such esters. The compounds of this invention are no exception in this respect, and can be effectively administered as an ester, formed on the hydroxy groups, just as one skilled in pharmaceutical chemistry would expect. It is possible, as has long been known in pharmaceutical chemistry, to adjust the rate or duration of action of the compound by appropriate choices of ester groups.

Certain ester groups are preferred as constituents of the compounds of this invention. The estrogen agonists / antagonists and cGMP elevators including the compounds of formula I or IA may contain ester groups at various positions as defined herein above, where these groups are represented as -COOR, R is C₁ -C₁₄ alkyl, C₁ -C₃ chloroalkyl, C₁ -C₃ fluoroalkyl, C₅ -C₇ cycloalkyl, phenyl, or phenyl mono- or disubstituted with C₁ -C₄ alkyl, C₁-C₄ alkoxy, hydroxy, nitro, chloro, fluoro or tri(chloro or fluoro)methyl.

As used herein, the term "effective amount" means an amount of compound of the compositions, kits and methods of the present invention that is capable of treating the described pathological conditions. The specific dose of a compound administered according to this invention will, of course, be determined by the particular circumstances surrounding the case including, for example, the compound administered, the route of administration, the state of being of the patient, and the severity of the pathological condition being treated.

The dose of a compound of this invention to be administered to a subject is rather widely variable and subject to the judgement of the attending physician. It should be noted that it may be necessary to adjust the dose of a compound when it is administered in the form of a salt, such as a laureate, the salt forming moiety of which has an appreciable molecular weight.

The following dosage amounts and other dosage amounts set forth elsewhere in this description and in the appendant claims are for an average human subject having a weight of about 65 kg to about 70 kg. The skilled practitioner will readily be able to determine the dosage amount required for a subject whose weight falls outside the 65 kg to 70 kg range, based upon the medical history of the subject and the presence of diseases, e.g., diabetes, in the subject. All doses set forth herein, and in the appendant claims, are daily doses of the free base form of the estrogen agonists / antagonists or cGMP elevators. Calculation of the dosage amount for other forms of the free base form such as salts or hydrates is easily accomplished by performing a simple ratio relative to the molecular weights of the species involved.

The general range of effective administration rates of the estrogen agonists / antagonists is from about 0.001 mg/day to about 200 mg/day. A preferred rate range is from about 0.010 mg/day to 100 mg/day. Of course, it is often practical to administer the daily dose of compound in portions, at various hours of the day. However, in any given case, the amount of compound administered will depend on such factors as the potency of the specific estrogen agonist/antagonist, the solubility of the compound, the formulation used and the route of administration.

Methods of formulation are well known in the art and are disclosed, for example, in Remington: The Science and Practice of Pharmacy, Mack Publishing Company, Easton, Pa., 19th Edition (1995). Pharmaceutical compositions for use in the present invention can be in the form of sterile, non-pyrogenic liquid solutions or suspensions, coated capsules, suppositories, lyophilized powders, transdermal patches or other forms known in the art.

Capsules are prepared by mixing the compound with a suitable diluent and filling the proper amount of the mixture in capsules. The usual diluents include inert powdered substances such as starch of many different kinds, powdered cellulose, especially crystalline and microcrystalline cellulose, sugars such as fructose, mannitol and sucrose, grain flours and similar edible powders.

Tablets are prepared by direct compression, by wet granulation, or by dry granulation. Their formulations usually incorporate diluents, binders, lubricants and disintegrators as well as the compound. Typical diluents include, for example, various types of starch, lactose, mannitol, kaolin, calcium phosphate or sulfate, inorganic salts such as sodium chloride and powdered sugar. Powdered cellulose derivatives are also useful. Typical tablet binders are substances such as starch, gelatin and sugars such as lactose, fructose, glucose and the like. Natural and synthetic gums are also convenient, including acacia, alginates, methylcellulose, polyvinylpyrrolidine and the like. Polyethylene glycol, ethylcellulose and waxes can also serve as binders.

A lubricant may be necessary in a tablet formulation to prevent the tablet and punches from sticking in the die. The lubricant is chosen from such slippery solids as talc, magnesium and calcium stearate, stearic acid and hydrogenated vegetable oils.

Tablet disintegrators are substances which facilitate the disintegration of a tablet to release a compound when the tablet becomes wet. They include starches, clays, celluloses, algins and gums, more particularly, corn and potato starches, methylcellulose, agar, bentonite, wood cellulose, powdered natural sponge, cation-exchange resins, alginic acid, guar gum, citrus pulp and carboxymethylcellulose, for example, may be used as well as sodium lauryl sulfate.

Tablets are often coated with sugar as a flavorant and sealant, or with film-forming protecting agents to modify the dissolution properties of the tablet. The compounds may also be formulated as chewable tablets, by using large amounts of pleasant-tasting substances such as mannitol in the formulation, as is now well-established in the art.

When it is desired to administer a compound as a suppository, the typical bases may be used. Cocoa butter is a traditional suppository base, which may be modified by addition of waxes to raise its melting point slightly. Water-miscible suppository bases comprising, particularly, polyethylene glycols of various molecular weights are in wide use.

The effect of the compounds may be delayed or prolonged by proper formulation. For example, a slowly soluble pellet of the compound may be prepared and incorporated in a tablet or capsule. The technique may be improved by making pellets of several different dissolution rates and filling capsules with a mixture of the pellets. Tablets or capsules may be coated with a film which resists dissolution for a predictable period of time. Topical formulations may be designed to yield delayed and/or prolonged percutaneous absorption of a compound. Even the parenteral preparations may be made long-acting, by dissolving or suspending the compound in oily or emulsified vehicles which allow it to disperse only slowly in the serum.

As used herein, the term "effective amount" means an amount of compound of the methods of the present invention that is capable of treating the pathological condition(s). The specific dose of a compound administered according to this invention will, of course, be determined by the particular circumstances surrounding the case including, for example, the compound administered, the route of administration, the state of being of the subject, and the severity of the pathological condition being treated.

Based on a reading of the present description and claims, certain modifications to the compositions and methods described herein will be apparent to one of ordinary skill in the art. The claims appended hereto are intended to encompass these modifications.

### EXAMPLES

### Example 1: Estrogen Receptor Binding.

Estrogen and estrogen agonist / antagonist binding affinity was measured by the following protocol:
cDNA cloning of human ERα: The coding region of human ERα was cloned by RT-PCR from human breast cancer cell mRNA using Expand^{™} High Fidelity PCR System according to manufacturer's instructions (Boehringer-Mannheim, Indianapolis, IN). PCR products were cloned into pCR2.1 TA Cloning Kit (Invitrogen, Carlsbad, CA) and sequenced. Each receptor-coding region was subcloned into the mammalian expression vector pcDNA3 ((Invitrogen, Carlsbad, CA).
Mammalian cell expression. Receptor proteins were overexpressed in 293T cells. These cells, derived from HEK293 cells (ATCC, Manassas, VA), have been engineered to stably express large T antigen and can therefore replicate plasmids containing a SV40 origin of replication to high copy numbers. 293T cells were transfected with either hERα-pcDNA3 or hERβ-pcDNA3 using lipofectamine as described by the manufacturer (Gibco/BRL, Bethesda, MD). Cells were harvested in phosphate buffered saline (PBS) with 0.5 mM EDTA at 48 h post-transfection. Cell pellets were washed once with PBS/EDTA. Whole cell lysates were prepared by homogenization in TEG buffer (50 mM Tris pH 7.4, 1.5 mM EDTA, 50 mM NaCl, 10% glycerol, 5 mM DTT, 5 µg/ml aprotinin, 10 µg/ml leupeptin, 0.1 mg/ml Pefabloc) using a dounce homogenizor. Extracts were centrifuged at 100,000 x g for 2 h at 4°C and supernatants were collected. Total protein concentrations were determined using BioRad reagent (BioRad, Hercules, CA).
Competition binding assay. The ability of various compounds to inhibit [³H]-estradiol binding was measured by a competition binding assay using dextran-coated charcoal as has been described (Leake RE, Habib F 1987 Steroid hormone receptors: assay and characterization. In: B. Green and R.E. Leake (eds). Steroid Hormones a Practical Approach. IRL Press Ltd, Oxford. 67-92.) 293T cell extracts expressing either hERα or hERβ were incubated in the presence of increasing concentrations of compound to be tested and a fixed concentration of [³H]-estradiol (141 µCi/mmol, New England Nuclear, Boston, MA) in 50 mM TrisHCl pH 7.4, 1.5 mM EDTA, 50 mM NaCl, 10% glycerol, 5 mM DTT, 0.5 mg/mL β-lactoglobulin in a final volume of 0.2 mL. All compounds to be tested were dissolved in dimethylsulfoxide. The final concentration of receptor was 50 pM with 0.5 nM [³H]-estradiol. After 16 h at 4°C, dextran-coated charcoal (20 µL) was added. After 15 min at room temperature the charcoal was removed by centrifugation and the radioactive ligand present in the supernatant was measured by scintillation counting. All reagents were obtained from Sigma (St. Louis, MO) unless otherwise indicated.

The binding affinity of (-)-cis-6-phenyl-5-[4-(2-pyrrolidin-1-yl-ethoxy)-phenyl]-5,6,7,8-tetrahydro-naphthalene-2=ol (PPTN) and 17β-estradiol were measured using recombinant human estrogen receptor (ER). Figure 1 shows the results of the binding experiment in which the binding of PPTN was found to be similar to that of 17β-estradiol.

### Example 2: Inhibition of In Vitro Human Breast Tumor Cell Growth.

The *in vitro* antiproliferative effects of (-)-cis-6-phenyl-5-[4-(2-pyrrolidin-1-yl-ethoxy)-phenyl]-5,6,7,8-tetrahydro-naphthalene-2-ol (PPTN) were tested using two types of human breast cancer cell lines: first, MCF-7 cells, which contain ER as well as progesterone receptors (PgR), and second, MDA-MB-231 cells, which lack ER and PgR, and enable the determination of an effect that is independent of the ER mechanism. The effect of PPTN on the growth of these different cell lines was determined by incubation of the cells with various compound concentrations for 6 days.

The antiproliferative effects were then determined by direct cell counts. PPTN inhibited the growth of the ER-positive cell line MCF-7. The IC₅₀ for growth inhibition was approximately 3 to 5 x 10⁻¹¹ M. In MDA-MB-231, ER-negative cell lines, the compound did not inhibit cell proliferation. These results demonstrate that growth inhibition was ER-specific and not due to cytotoxicity since the compound had no measurable effect on the ER-negative cell line.

### Example 3: Measurement of sexual functioning in post-menopausal women.

Sexual functioning and satisfaction in post-menopausal women is evaluated in a 52 week, placebo-controlled clinical study using a modified Women's Health Questionnaire (WHQ) as the measurement technique. Prior to the commencement in the study, post-menopausal women are divided into two groups of between 5 and 100 women in each group. One group is a placebo control group. The other group is a test group that receives a pharmaceutical composition containing an estrogen agonist / antagonist. At the start of the study, all participants in both groups complete a WHQ. Participants in the control group receive a daily placebo composition. Participants in the test group receive a composition containing an estrogen agonist / antagonist. At the end of the study, participants in both groups again complete the WHQ. The results of the WHQ from the control group and the test group are then compared.

The Women's Health Questionnaire (WHQ) provides a detailed examination of minor psychological and somatic symptoms experienced by peri- and postmenopausal women (Hunter M., et al., Maturitas; 8: 217, 1986). The WHQ is well documented in terms of reliability and validity. The questionnaire has 36 questions rated on four-point scales. The higher the score, the more pronounced is the distress and dysfunction. The 36 items combine into nine factors describing somatic symptoms, depressed mood, cognitive difficulties, anxiety/fear, sexual functioning, vasomotor symptoms, sleep problems, menstrual symptoms and attraction. The modified Woman's Health Questionnaire for this study contains specific questions regarding female sexual dysfunction including hypoactive sexual desire disorder, sexual arousal disorder, dyspareunia and vaginismus.

## Claims

1. The use of an estrogen agonist / antagonist of the following formula (I): 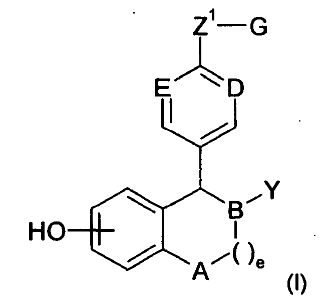 wherein:
A is selected from CH₂ and NR;
B, D and E are independently selected from CH and N;
Y is
(a) phenyl, optionally substituted with 1-3 substituents independently selected from R⁴;
(b) naphthyl, optionally substituted with 1-3 substituents independently selected from R⁴;
(c) C₃-C₈ cycloalkyl, optionally substituted with 1-2 substituents independently selected from R⁴;
(d) C₃-C₈ cycloalkenyl, optionally substituted with 1-2 substituents independently selected from R⁴;
(e) a five membered heterocycle containing up to two heteroatoms selected from the group consisting of -O-, -NR²- and -S(O)ₙ-, optionally substituted with 1-3 substituents independently selected from R⁴;
(f) a six membered heterocycle containing up to two heteroatoms selected from the group consisting of -O-, -NR²- and -S(O)ₙ- optionally substituted with 1-3 substituents independently selected from R⁴; or
(g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
(a) -(CH₂)ₚW(CH₂)_{q}-;
(b) -O(CH₂)ₚCR⁵R⁶-;
(c) -O(CH₂)ₚW(CH₂)_{q}-;
(d) -OCHR²CHR³-; or
(e) -SCHR²CHR³-;
G is
(a) -NR⁷R⁸;
(b) 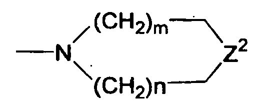 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
(c) a bicyclic amine containing five to twelve carbon atoms, either bridged or fused and optionally substituted with 1-3 substituents independently selected from R⁴; or
Z¹ and G in combination may be 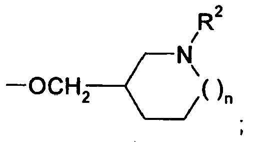
Wis
(a) -CH₂-;
(b) -CH=CH-;
(c) -O-;
(d) -NR²-;
(e) -S(O)ₙ-;
(f) 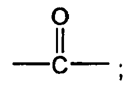
(g) -CR²(OH)-;
(h) -CONR²-;
(i) -NR²CO-;
(j) 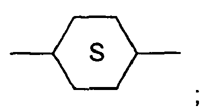 or
(k) -C≡C-;
R is hydrogen or C₁-C₆ alkyl;
R² and R³ are independently
(a) hydrogen; or
(b) C₁-C₄ alkyl;
R⁴ is
(a) hydrogen;
(b) halogen;
(c) C₁-C₆ alkyl;
(d) C₁-C₄ alkoxy;
(e) C₁-C₄ acyloxy;
(f) C₁-C₄ alkylthio;
(g) C₁-C₄ alkylsulfinyl; '
(h) C₁-C₄ alkylsulfonyl;
(i) hydroxy (C₁-C₄)alkyl;
(j) aryl (C₁-C₄)alkyl;
(k) -CO₂H;
(l) -CN;
(m) -CONHOR;
(n) -SO₂NHR;
(o) -NH₂;
(p) C₁-C₄ alkylamino;
(q) C₁-C₄ dialkylamino;
(r) -NHSO₂R;
(s) -NO₂;
(t) -aryl; or
(u) -OH;
R⁵ and R⁶ are independently C₁-C₈ alkyl or together form a C₃-C₁₀ carbocyclic ring;
R⁷ and R⁸ are independently
(a) phenyl;
(b) a C₃-C₁₀ carbocyclic ring, saturated or unsaturated;
(c) a C₃-C₁₀ heterocyclic ring containing up to two heteroatoms, selected from -O-, -N- and -S-;
(d) H;
(e) C₁-C₆ alkyl; or
(f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
e is 0, 1 or 2;
m is 1, 2 or 3;
n is 0, 1 or 2;
p is 0, 1, 2 or 3;
q is 0, 1, 2 or 3;
or an optical or geometric isomer thereof; or a pharmaceutically acceptable salt, N-oxide, ester, or quaternary ammonium salt thereof;
in the manufacture of a medicament for treating sexual arousal disorder, dyspareunia and vaginismus.

2. The use according to claim 1 wherein said estrogen agonist / antagonist is a compound of formula (IA): 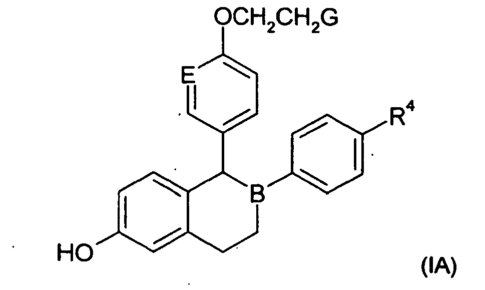 wherein G is 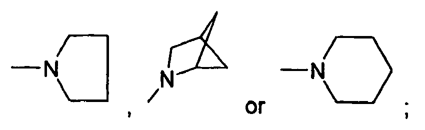 R⁴ is H, OH, F, or Cl; and B and E are independently selected from CH and N or an optical or geometric isomer thereof; or a pharmaceutically acceptable salt, N-oxide, ester, or a quaternary ammonium salt thereof.

3. The use according to claim 2 wherein said estrogen agonist / antagonist is (-)-cis-6-phenyl-5-[4-(2-pyrrolidin-1-yl-ethoxy)-phenyl]-5,6,7,8-tetrahydronaphthalene-2-ol or an optical or geometric isomer thereof; a pharmaceutically acceptable salt, N-oxide, ester, or a quaternary ammonium salt thereof.

4. The use according to claim 3 wherein said estrogen agonist / antagonist is in the form of a D-tartrate salt.

5. The use according to any preceding claim further comprising co-administrering a cyclic guanosine 3',5'-monophosphate elevator.

6. The use according to claim 5 wherein said cyclic guanosine 3',5'-monophosphate elevator is a PDEᵥ phosphodiesterase inhibitor.

7. The use according to claim 6 further comprising co-administrering 1-[[3-(6,7-dihydro-1-methyl-7-oxo-3-propyl-1H-pyrazolo[4,3-d]pyrimidin-5-yl)-4-ethoxyphenyl]sufonyl]-4-methylpiperazine citrate salt.

8. The use according to any of the preceding claims wherein the condition to be treated is sexual arousal disorder.

9. The use according to any of the preceding claims wherein the condition to be treated is selected from dyspareunia and vaginismus.

10. A pharmaceutical composition comprising:
(a) an estrogen agonist / antagonist as defined in any one of claims 1 to 4,
(b) a cyclic guanosine 3',5'-monophosphate elevator, and
(c) a pharmaceutically acceptable carrier.

11. A pharmaceutical composition according to claim 10 wherein said cyclic guanosine 3',5'-monophosphate elevator is a PDEᵥ phosphodiesterase inhibitor.

12. A pharmaceutical composition as in claim 11 wherein said PDEᵥ phosphodiesterase inhibitor is 1-[[3-(6,7-dihydro-1-methyl-7-oxo-3-propyl-1H-pyrazolo[4,3-d]pyrimidin-5-yl)-4-ethoxy-phenyl]sufonyl]-4-methylpiperazine citrate salt.

## Patentansprüche

1. Verwendung eines Östrogenagonisten/antagonisten der im folgenden angegebenen Formel (I): 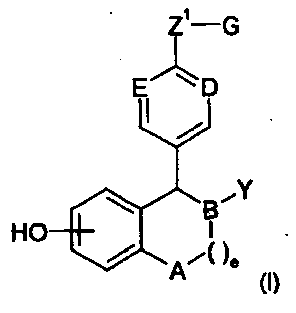 worin:
A aus CH₂ und NR ausgewählt ist;
B, D und E unabhängig voneinander aus CH und N ausgewählt sind;
Y für
(a) Phenyl, das optional mit 1-3 Substituenten substituiert ist, die unabhängig voneinander aus R⁴ ausgewählt sind;
(b) Naphthyl, das optional mit 1-3 Substituenten substituiert ist, die unabhängig voneinander aus R⁴ ausgewählt sind;
(c) C₃-C₈-Cycloalkyl, das optional mit 1-2 Substituenten substituiert ist, die unabhängig voneinander aus R⁴ ausgewählt sind;
(d) C₃-C₈-Cycloalkenyl, das optional mit 1-2 Substituenten substituiert ist, die unabhängig voneinander aus R⁴ ausgewählt sind;
(e) einen fünfgliedrigen Heterocyclus, der bis zu zwei Heteroatome enthält, die aus der Gruppe von -O-, -NR²- und -S(O)ₙ- ausgewählt sind, der optional mit 1-3 Substituenten substituiert ist, die unabhängig voneinander aus R⁴ ausgewählt sind;
(f) einen sechsgliedrigen Heterocyclus, der bis zu zwei Heteroatome enthält, die aus der Gruppe von -O-, -NR²- und -S(O)ₙ- ausgewählt sind, der optional mit 1-3 Substituenten substituiert ist, die unabhängig voneinander aus R⁴ ausgewählt sind; oder
(g) ein bicyclisches Ringsystem, das aus einem an einen Phenylring kondensierten fünf- oder sechsgliedrigen heterocyclischen Ring besteht, wobei der heterocyclische Ring bis zu zwei Heteroatome enthält, die aus der Gruppe von -O-, -NR²- und -S(O)ₙ- ausgewählt sind, das optional mit 1-3 Substituenten substituiert ist, die unabhängig voneinander aus R⁴ ausgewählt sind, steht;
Z¹ für
(a) -(CH₂)ₚW(CH₂)_{q}-;
(b) -O(CH₂)ₚCR⁵R⁶-;
(c) -O(CH₂)ₚW(CH₂)_{q}-;
(d) -OCHR²CHR³-; oder
(e) -SCHR²CHR³- steht;
G für
(a) -NR⁷R⁸;
(b) 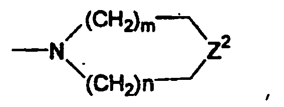 worin n 0, 1 oder 2 ist; m 1, 2 oder 3 ist; Z² -NH-, -O-, -S- oder -CH₂- ist; das optional an benachbarten Kohlenstoffatomen mit einem oder zwei Phenylringen kondensiert ist und optional unabhängig voneinander anm Kohlenstoff mit einem bis drei Substituenten substituiert ist und optional unabhängig voneinander am Stickstoff mit einem chemisch geeigneten Substituenten substituiert ist, die aus R⁴ ausgewählt sind; oder
(c) ein bicyclisches Amin, das 5 bis 12 Kohlenstoffatome enthält, das entweder verbrückt oder kondensiert ist und optional mit 1-3 Substituenten substituiert ist, die unabhängig voneinander aus R⁴ ausgewählt sind, steht; oder
Z¹ und G in Kombination für 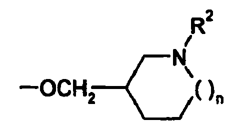 stehen können;
W für
(a) -CH₂-;
(b) -CH=CH-;
(c) -O-;
(d) -NR²-;
(e) -S(O)ₙ-;
(f) 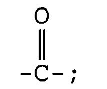
(g) -CR²(OH)-;
(h) -CONR²-;
(i) -NR²CO- ;
(j) 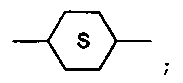 oder
(k) -C≡C- steht;
R für Wasserstoff oder C₁-C₆-Alkyl steht;
R² und R³ unabhängig voneinander für
(a) Wasserstoff oder
(b) C₁-C₄-Alkyl stehen;
R⁴ für
(a) Wasserstoff;
(b) Halogen;
(c) C₁-C₆-Alkyl ;
(d) C₁-C₄-Alkoxy;
(e) C₁-C₄-Acyloxy;
(f) C₁-C₄-Alkylthio;
(g) C₁-C₄-Alkylsulfinyl;
(h) C₁-C₄-Alkylsulfonyl;
(i) Hydroxy(C₁-C₄) alkyl;
(j) Aryl(C₁-C₄)alkyl;
(k) -CO₂H;
(l) -CN;
(m) -CONHOR;
(n) -SO₂NHR;
(o) -NH₂;
(p) C₁-C₄-Alkylamino;
(q) C₁-C₄-Dialkylamino;
(r) -NHSO₂R;
(s) -NO₂;
(t) -Aryl; oder
(u) -OH steht;
R⁵ und R⁶ unabhängig voneinander für C₁-C₈-Alkyl stehen oder zusammen einen carbocyclischen C₃-C₁₀-Ring bilden; R⁷ und R⁸ unabhängig voneinander für
(a) Phenyl;
(b) einen gesättigten oder ungesättigten carbocyclischen C₃-C₁₀-Ring;
(c) einen heterocyclischen C₃-C₁₀-Ring, der bis zu zwei Heteroatome enthält, die aus -O-, -N- und -S-ausgewählt sind;
(d) H;
(e) C₁-C₆-Alkyl stehen oder
(f) einen 3- bis 8-gliedrigen stickstoffhaltigen Ring mit R⁵ oder R⁶ bilden;
R⁷ und R⁸ in entweder linearer oder Ringform optional mit bis zu drei Substituenten substituiert sein können, die unabhängig voneinander aus C₁-C₆-Alkyl, Halogen, Alkoxy, Hydroxy und Carboxy ausgewählt sind;
ein von R⁷ und R⁸ gebildeter Ring optional an einen Phenylring kondensiert sein kann;
e 0, 1 oder 2 ist;
m 1, 2 oder 3 ist;
n 0, 1 oder 2 ist;
p 0, 1, 2 oder 3 ist;
q 0, 1, 2 oder 3 ist;
oder eines optischen oder geometrischen Isomers desselben oder eines pharmazeutisch akzeptablen Salzes, N-Oxids, Esters oder quaternären Ammoniumsalzes derselben;
bei der Herstellung eines Medikaments zur Behandlung von sexueller Erregungsstörung, Dyspareunie und Vaginismus.

2. Verwendung nach Anspruch 1, wobei der Östrogenagonist/antagonist eine Verbindung der Formel (IA) : 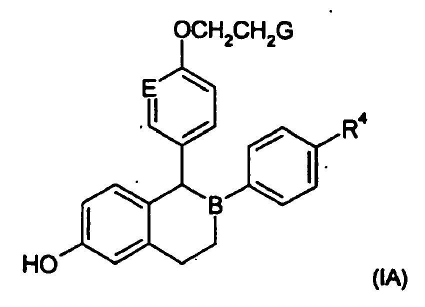 worin G für 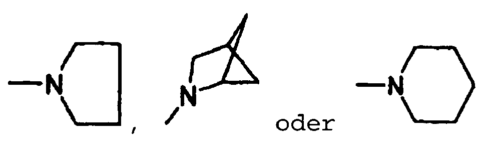 steht;
R⁴ für H, OH, F oder Cl steht;
und B und E unabhängig voneinander aus CH und N ausgewählt sind;
oder ein optisches oder geometrisches Isomer derselben oder ein pharmazeutisch akzeptables Salz, N-Oxid, ein pharmazeutisch akzeptabler Ester oder ein pharmazeutisch akzeptables quaternäres Ammoniumsalz derselben ist.

3. Verwendung nach Anspruch 2, wobei der Östrogenagonist/antagonist (-)-cis-6-Phenyl-5-[4-(2-pyrrolidin-1-yl-ethoxy)-phenyl]-5,6,7,8-tetrahydronaphthalin-2-ol oder ein optisches oder geometrisches Isomer desselben, ein pharmazeutisch akzeptables Salz, N-Oxid, ein pharmazeutisch akzeptabler Ester oder ein pharmazeutisch akzeptables quaternäres Ammoniumsalz desselben ist.

4. Verwendung nach Anspruch 3, wobei der Östrogenagonist/antagonist in der Form eines D-Tartratsalzes vorliegt.

5. Verwendung nach einem der vorhergehenden Ansprüche, die ferner die Co-Verabreichung eines cyclisches Guanosin-3',5'-monophosphat erhöhenden Mittels umfasst.

6. Verwendung nach Anspruch 5, wobei das cyclisches Guanosin-3',5'-monophosphat erhöhende Mittel ein PDE_{V}-Phosphodiesteraseinhibitor ist.

7. Verwendung nach Anspruch 6, die ferner die Co-Verabreichung des 1-[[3-(6,7-Dihydro-1-methyl-7-oxo-3-propyl-1H-pyrazolo[4,3-d]pyrimidin-5-yl)-4-ethoxyphenyl]sulfonyl]-4-methylpiperazin-citratsalzes umfasst.

8. Verwendung nach einem der vorhergehenden Ansprüche, wobei der zu behandelnde Zustand eine sexuelle Erregungsstörung ist.

9. Verwendung nach einem der vorhergehenden Ansprüche, wobei der zu behandelnde Zustand aus Dyspareunie und Vaginismus ausgewählt ist.

10. Pharmazeutische Zusammensetzung, die umfasst:
(a) einen Östrogenagonisten/antagonisten gemäß der Definition in einem der Ansprüche 1 bis 4,
(b) ein cyclisches Guanosin-3',5'-monophosphat erhöhendes Mittel und
(c) einen pharmazeutisch akzeptablen Träger.

11. Pharmazeutische Zusammensetzung nach Anspruch 10, wobei das cyclisches Guanosin-3',5'-monophosphat erhöhende Mittel ein PDE_{V}-Phosphodiesteraseinhibitor ist.

12. Pharmazeutische Zusammensetzung nach Anspruch 11, wobei der PDE_{V}-Phosphodiesteraseinhibitor ein 1-[[3-(6,7-Dihydro-1-methyl-7-oxo-3-propyl-1H-pyrazolo[4,3-d]pyrimidin-5-yl)-4-ethoxyphenyl]sulfonyl]-4-methylpiperazin-citratsalz ist.

## Revendications

1. Utilisation d'un agoniste/antagoniste des oestrogènes de formule (I) suivante : 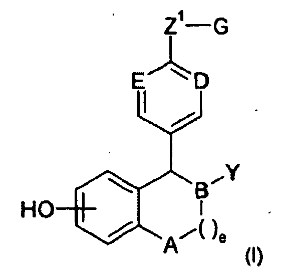 dans laquelle :
A est sélectionné parmi CH₂ et NR ;
B, D et E sont indépendamment sélectionnés parmi CH et N ;
Y représente
(a) phényle, facultativement substitué par 1-3 substituants indépendamment sélectionnés parmi R⁴ ;
(b) naphtyle, facultativement substitué par 1-3 substituants indépendamment sélectionnés parmi R⁴ ;
(c) cycloalkyle en C₃-C₈, facultativement substitué par 1-2 substituants indépendamment sélectionnés parmi R⁴ ;
(d) cycloalkényle en C₃-C₈, facultativement substitué par 1-2 substituants indépendamment sélectionnés parmi R⁴ ;
(e) un hétérocycle à cinq éléments contenant jusqu'à deux hétéroatomes sélectionnés dans le groupe consistant en -O-, -NR²- et -S(O)ₙ-, facultativement substitué par 1-3 substituants indépendamment sélectionnés parmi R⁴ ;
(f) un hétérocycle à six éléments contenant jusqu'à deux hétéroatomes sélectionnés dans le groupe consistant en -O-, -NR²- et -S(O)ₙ-, facultativement substitué par 1-3 substituants indépendamment sélectionnés parmi R⁴ ; ou
(g) un système de noyau bicyclique consistant en un noyau hétérocyclique à cinq ou six éléments condensé à un noyau phényle, ledit noyau hétérocyclique contenant jusqu'à deux hétéroatomes sélectionnés dans le groupe consistant en -O-, -NR²- et -S(O)ₙ-, facultativement substitué par 1-3 substituants indépendamment sélectionnés parmi R⁴ ;
Z¹ représente
(a) -(CH₂)ₚW(CH₂)_{q}- ;
(b) -O(CH₂)ₚCR⁵R⁶- ;
(c) -O(CH₂)ₚW(CH₂)_{q}- ;
(d) -OCHR²CHR³- ; ou
(e) -SCHR²CHR³- ;
G représente
(a) -NR⁷R⁸ ;
(b) 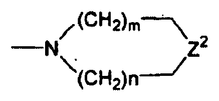 où n représente 0, 1 ou 2 ; m représente 1, 2 ou 3 ; Z² représente -NH-, -O-, -S- ou -CH₂- ; facultativement condensé sur des atomes de carbone adjacents de l'un ou des deux noyaux phényle et, facultativement et indépendamment substitué sur le carbone par de un à trois substituants et, facultativement et indépendamment sur l'azote par un substituant chimiquement convenable sélectionné parmi R⁴ ; ou
(c) une amine bicyclique contenant de cinq à douze atomes de carbone, soit pontée soit condensée et facultativement substituée par 1-3 substituants indépendamment sélectionnés parmi R⁴ ; ou
Z¹ et G, en combinaison, peuvent représenter : 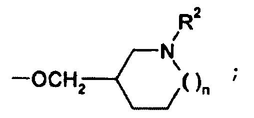
W représente :
(a) -CH₂- ;
(b) -CH=CH- ;
(c) -O- ;
(d) -NR²- ;
(e) S(O)ₙ-;
(f) 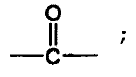
(g) -CR²(OH)-;
(h) -CONR²- ;
(i) -NR²CO- ;
(j) 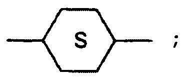 ou
(k) -C ≡ C- ;
R représente l'hydrogène ou alkyle en C₁-C₆ ;
R² et R³ représentent indépendamment
(a) l'hydrogène ; ou
(b) alkyle en C₁-C₄ ;
R⁴ représente :
(a) l'hydrogène ;
(b) un halogène ;
(c) alkyle en C₁-C₆ ;
(d) alcoxy en C₁-C₄ ;
(e) acyloxy en C₁-C₄ ;
(f) (alkyle en C₁-C₄)thio ;
(g) (alkyle en C₁-C₄) sulfinyle ;
(h) (alkyle en C₁-C₄)sulfonyle ;
(i) (hydroxy) alkyle en C₁-C₄ ;
(j) (aryl)alkyle en C₁-C₄ ;
(k) -CO₂H ;
(l) -CN ;
(m) -CONHOR ;
(n) -SO₂NHR ;
(o) -NH₂ ;
(p) (alkyle en C₁-C₄)amino ;
(q) (dialkyle en C₁-C₄)amino ;
(r) -NHSO₂R ;
(s) -NO₂ ;
(t) -aryle ; ou
(u) -OH ;
R⁵ et R⁶ représentent indépendamment alkyle en C₁-C₈ ou forment ensemble un noyau carbocyclique en C₃-C₁₀;
R⁷ et R⁸ représentent indépendamment
(a) phényle ;
(b) un noyau carbocyclique en C₃-C₁₀, saturé ou insaturé ;
(c) un noyau hétérocyclique en C₃-C₁₀ contenant jusqu'à deux hétéroatomes, sélectionnés parmi -O-, -N- et -S- ;
(d) H ;
(e) alkyle en C₁-C₆ ; ou
(f) forment, avec R⁵ ou R⁶, un noyau ayant de 3 à 8 éléments et contenant de l'azote ;
R⁷ et R⁸, sous forme linéaire ou cyclique, peuvent être facultativement substitués par jusqu'à trois substituants indépendamment sélectionnés parmi alkyle en C₁-C₆, halogéno, alcoxy, hydroxy et carboxy ;
un noyau formé par R⁷ et R⁸ peut être facultativement condensé à un noyau phényle ;
e représente 0, 1 ou 2 ;
m représente 1, 2 ou 3 ;
n représente 0, 1 ou 2 ;
p représente 0, 1, 2 ou 3 ;
q représente 0, 1, 2 ou 3 ;
ou un isomère optique ou géométrique correspondant ; ou un sel, N-oxyde, ester ou sel d'ammonium quaternaire pharmaceutiquement acceptable correspondant ;
dans la fabrication d'un médicament pour le traitement d'un trouble de l'excitation sexuelle, de la dyspareunie et du vaginisme.

2. Utilisation selon la revendication 1, dans laquelle ledit agoniste/antagoniste des oestrogènes est un composé de formule (IA) : 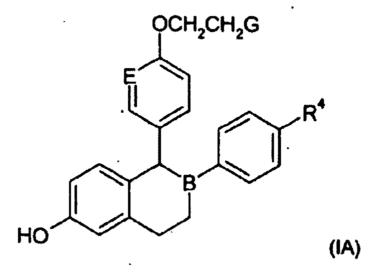 dans laquelle G représente 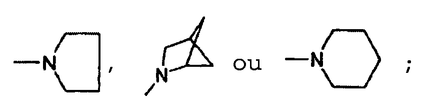 R⁴ représente H, OH, F ou Cl ; et B et E sont indépendamment sélectionnés parmi CH et N ou un isomère optique ou géométrique de ceux-ci ; ou un sel, N-oxyde, ester ou sel d'ammonium quaternaire pharmaceutiquement acceptable correspondant.

3. Utilisation selon la revendication 2, dans laquelle ledit agoniste/antagoniste des oestrogènes est le (-)-cis-6-phényl-5-[4-(2-pyrrolidin-1-yl-éthoxy)-phényl]-5,6,7,8-tétrahydro-naphtalén-2-ol ou un isomère optique ou géométrique de celui-ci ; ou un sel, N-oxyde, ester ou sel d'ammonium quaternaire pharmaceutiquement acceptable correspondant.

4. Utilisation selon la revendication 3, dans laquelle l'agoniste/antagoniste des oestrogènes est sous la forme d'un D-tartrate.

5. Utilisation selon l'une quelconque des revendications précédentes, comprenant la co-administration d'un élévateur de la guanosine 3',5'-monophosphate cyclique.

6. Utilisation selon la revendication 5, dans laquelle ledit élévateur de la guanosine 3',5'-monophosphate cyclique est un inhibiteur de la PDEᵥ phosphodiestérase.

7. Utilisation selon la revendication 6, comprenant en outre la co-administration de citrate de 1-[[3-(6,7-dihydro-1-méthyl-7-oxo-3-propyl-1H-pyrazolo [4,3-d]pyrimidin-5-yl)-4-éthoxy-phényl]sulfonyl]-4-méthyl-pipérazine.

8. Utilisation selon l'une quelconque des revendications précédentes, dans laquelle l'état à traiter est un trouble de l'excitation sexuelle.

9. Utilisation selon l'une quelconque des revendications précédentes, dans laquelle l'état à traiter est sélectionné parmi la dyspareunie et le vaginisme.

10. Composition pharmaceutique comprenant :
(a) un agoniste/antagoniste des oestrogènes tel que défini dans l'une quelconque des revendications 1 à 4,
(b) un élévateur de la guanosine 3',5'-monophosphate cyclique, et
(c) un support pharmaceutiquement acceptable.

11. Composition pharmaceutique selon la revendication 10, dans laquelle ledit élévateur de la guanosine 3',5'-monophosphate cyclique est un inhibiteur de la PDEᵥ, phosphodiestérase.

12. Composition pharmaceutique selon la revendication 11, dans laquelle ledit inhibiteur de la PDEᵥ phosphodiestérase est le citrate de 1-[[3-(6,7-dihydro-1-méthyl-7-oxo-3-propyl-1H-pyrazolo [4,3-d]pyrimidin-5-yl)-4-éthoxy-phényl]sulfonyl]-4-méthyl-pipérazine.
